# EUROPEAN PATENT APPLICATION

(11) **EP 3 848 464 A1**
(43) Date of publication of application: **14.07.2021**
(21) Application number: 19830148.3
(22) Date of filing: 03.07.2019
(51) Int. Cl.: C12N 15/82, C12N 9/22, C12N 15/70, A01H 5/00, A01H 6/54

(54) **METHOD FOR SITE-SPECIFIC MUTAGENESIS OF MEDICAGO SATIVA GENE BY USING CRISPR/CAS9 SYSTEM**

(30) Priority: 04.07.2018 CN 201810724589
(71) Applicant: Guangdong Sanjie Forage Biotechnology Co., Ltd, Guangzhou, Guangdong 510623 (CN)
(72) Inventor: CHEN, Haitao, Guangzhou, Guangdong 510507 (CN); WANG, Wen, Guangzhou, Guangdong 510507 (CN); XIE, Xiongping, Guangzhou, Guangdong 510507 (CN); QIU, Qiang, Guangzhou, Guangdong 510507 (CN); SHANG, Zhanhuan, Guangzhou, Guangdong 510507 (CN); SU, Kexian, Guangzhou, Guangdong 510507 (CN); HE, Hui, Guangzhou, Guangdong 510507 (CN)
(74) Representative: Sonn & Partner Patentanwälte
(86) International application number: PCT/CN2019/094630
(87) International publication number: WO 2020/007331

(57) **Abstract**

A method for site-specific mutagenesis of *Medicago sativa* genes by using a CRISPR/Cas9 system. The method comprises: first constructing a binary expression vector MsCRISPR/Cas9 that can be used for transforming *Medicago sativa* by *Agrobacterium tumefaciens;* then designing a target site for a target gene, and ligating the DNA fragment containing the guide sequence of the target site into MsCRISPR/Cas9 to construct a vector MsCRISPR/Cas9::*target*; and then transforming the *Medicago sativa* by *Agrobacterium tumefaciens,* and generating, by screening, a mutant transformed plant with the target gene mutated. According to the method, an MtU6 promoter is used for driving sgRNA transcription in the *Medicago sativa.*

### SEQUENCE LISTING

<110>   GUANGDONG SANJIE FORAGE BIOTECHNOLOGY CO., LTD

<120>   Method for site-specific mutagenesis of Medicago sativa gene by
        using CRISPR/Cas9 system

<130>   GWPCTP202011375

<160>   4

<170>   PatentIn version 3.5

<210>   1
<211>   14951
<212>   DNA
<213>   artificial sequence

<220>

<223>    binary expression vector MsCRISPR/Cas9

<400>    1

aacgtcgcca gggcgtaggt ggtcaagcat cctggccagc tccgggcggt cgcgcctggt          60

gccggtgatc ttctcggaaa acagcttggt gcagccggcc gcgtgcagtt cggcccgttg          120

gttggtcaag tcctggtcgt cggtgctgac gcgggcatag cccagcaggc cagcggcggc          180

gctcttgttc atggcgtaat gtctccggtt ctagtcgcaa gtattctact ttatgcgact          240

aaaacacgcg acaagaaaac gccaggaaaa gggcagggcg gcagcctgtc gcgtaactta          300

ggacttgtgc gacatgtcgt tttcagaaga cggctgcact gaacgtcaga agccgactgc          360

actatagcag cggaggggtt ggatcaaagt actttgatcc cgaggggaac cctgtggttg          420

gcatgcacat acaaatggac gaacggataa accttttcac gcccttttaa atatccgatt          480

attctaataa acgctctttt ctcttaggtt tacccgccaa tatatcctgt caaacactga          540

tagtttaatt cccgatctag taacatagat gacaccgcgc gcgataattt atcctagttt 600

gcgcgctata ttttgttttc tatcgcgtat taaatgtata attgcgggac tctaatcata 660

aaaacccatc tcataaataa cgtcatgcat tacatgttaa ttattacatg cttaacgtaa 720

ttcaacagaa attatatgat aatcatcgca agaccggcaa caggattcaa tcttaagaaa 780

ctttattgcc aaatgtttga acgatcgggg aaattcgagc tggtcacctg taattcacac 840

gtggtggtgg tggtggtggc tagcgttaac actagtcaga tctaccatgg tggactcctc 900

ttagaattcc cgatctagta acatagatga caccgcgcgc gataatttat cctagtttgc 960

gcgctatatt ttgttttcta tcgcgtatta aatgtataat tgcgggactc taatcataaa 1020

aacccatctc ataaataacg tcatgcatta catgttaatt attacatgct taacgtaatt 1080

caacagaaat tatatgataa tcatcgcaag accggcaaca ggattcaatc ttaagaaact 1140

ttattgccaa atgtttgaac gatcggggaa attcgagctc tatcgatcaa tcaggatcct 1200

tactttttct tttttgcctg gccggccttt ttcgtggccg ccggcctttt gtcgcctccc 1260

agctgagaca ggtcgatccg tgtctcgtac aggccggtga tgctctggtg gatcagggtg 1320

gcgtccagca cctctttggt gctggtgtac ctcttccggt cgatggtggt gtcaaagtac 1380

ttgaaggcgg caggggctcc cagattggtc agggtaaaca ggtggatgat attctcggcc 1440

tgctctctga tgggcttatc ccggtgcttg ttgtaggcgg acagcacttt gtccagatta 1500

gcgtcggcca ggatcactct cttggagaac tcgctgatct gctcgatgat ctcgtccagg 1560

tagtgcttgt gctgttccac aaacagctgt ttctgctcat tatcctcggg ggagcccttc 1620

agcttctcat agtggctggc caggtacagg aagttcacat atttggaggg cagggccagt 1680

tcgtttccct tctgcagttc gccggcagag gccagcattc tcttccggcc gttttccagc 1740

tcgaacaggg agtacttagg cagcttgatg atcaggtcct ttttcacttc tttgtagccc 1800

ttggcttcca gaaagtcgat gggattcttc tcgaagctgc ttctttccat gatggtgatc 1860

cccagcagct ctttcacact cttcagtttc ttggacttgc ccttttccac tttggccacc 1920

accagcacag aataggccac ggtggggctg tcgaagccgc cgtacttctt agggtcccag 1980

tccttctttc tggcgatcag cttatcgctg ttcctcttgg gcaggataga ctctttgctg 2040

aagccgcctg tctgcacctc ggtctttttc acgatattca cttggggcat gctcagcact 2100

ttccgcacgg tggcaaaatc ccggccctta tcccacacga tctccccggt ttcgccgttt 2160

gtctcgatca gaggccgctt ccggatctcg ccgttggcca gggtaatctc ggtcttgaaa 2220

aagttcatga tgttgctgta gaagaagtac ttggcggtag ccttgccgat ttcctgctcg 2280

ctcttggcga tcatcttccg cacgtcgtac accttgtagt cgccgtacac gaactcgctt 2340

tccagcttag ggtacttttt gatcagggcg gttcccacga cggcgttcag gtaggcgtcg 2400

tgggcgtggt ggtagttgtt gatctcgcgc actttgtaaa actggaaatc cttccggaaa 2460

tcggacacca gcttggactt cagggtgatc actttcactt cccggatcag cttgtcattc 2520

tcgtcgtact tagtgttcat ccgggagtcc aggatctgtg ccacgtgctt tgtgatctgc 2580

cgggtttcca ccagctgtct cttgatgaag ccggccttat ccagttcgct caggccgcct 2640

ctctcggcct tggtcagatt gtcgaacttt ctctgggtaa tcagcttggc gttcagcagc 2700

tgccgccagt agttcttcat cttcttcacg acctcttcgg agggcacgtt gtcgctcttg 2760

ccccggttct tgtcgcttct ggtcagcacc ttgttgtcga tggagtcgtc cttcagaaag 2820

ctctgaggca cgatatggtc cacatcgtag tcggacagcc ggttgatgtc cagttcctgg 2880

tccacgtaca tatcccgccc attctgcagg tagtacaggt acagcttctc gttctgcagc 2940

tgggtgtttt ccacggggtg ttctttcagg atctggctgc ccagctcttt gatgccctct 3000

tcgatccgct tcattctctc gcggctgttc ttctgtccct tctgggtggt ctggttctct 3060

ctggccattt cgatcacgat gttctcgggc ttgtgccggc ccatcacttt cacgagctcg 3120

tccaccacct tcactgtctg caggatgccc ttcttaatgg cggggctgcc ggccagattg 3180

gcaatgtgct cgtgcaggct atcgccctgg ccggacacct gggctttctg gatgtcctct 3240

ttaaaggtca ggctgtcgtc gtggatcagc tgcatgaagt ttctgttggc gaagccgtcg 3300

gacttcagga aatccaggat tgtcttgccg gactgcttgt cccggatgcc gttgatcagc 3360

ttccggctca gcctgcccca gccggtgtat ctccgccgct tcagctgctt catcactttg 3420

tcgtcgaaca ggtgggcata ggttttcagc cgttcctcga tcatctctct gtcctcaaac 3480

agtgtcaggg tcagcacgat atcttccaga atgtcctcgt tttcctcatt gtccaggaag 3540

tccttgtcct tgataatttt cagcagatcg tggtatgtgc ccagggaggc gttgaaccga 3600

tcttccacgc cggagatttc cacggagtcg aagcactcga ttttcttgaa gtagtcctct 3660

ttcagctgct tcacggtcac tttccggttg gtcttgaaca gcaggtccac gatggccttt 3720

ttctgctcgc cgctcaggaa ggcgggcttt ctcattccct cggtcacgta tttcactttg 3780

gtcagctcgt tatacacggt gaagtactcg tacagcaggc tgtgcttggg cagcaccttc 3840

tcgttgggca ggttcttatc gaagttggtc atccgctcga tgaagctctg ggcggaagcg 3900

cccttgtcca ccacttcctc gaagttccag ggggtgatgg tttcctcgct ctttctggtc 3960

atccaggcga atctgctgtt tcccctggcc agagggccca cgtagtaggg gatgcggaag 4020

gtcaggatct tctcgatctt ttcccggttg tccttcagga atgggtaaaa atcttcctgc 4080

cgccgcagaa tggcgtgcag ctctcccagg tggatctggt gggggatgct gccgttgtcg 4140

aaggtccgct gcttccgcag caggtcctct ctgttcagct tcacgagcag ttcctcggtg 4200

ccgtccatct tttccaggat gggcttgatg aacttgtaga actcttcctg gctggctccg 4260

ccgtcaatgt agccggcgta gccgttcttg ctctggtcga agaaaatctc tttgtacttc 4320

tcaggcagct gctgccgcac gagagctttc agcagggtca ggtcctggtg gtgctcgtcg 4380

tatctcttga tcatagaggc gctcaggggg gccttggtga tctcggtgtt cactctcagg 4440

atgtcgctca gcaggatggc gtcggacagg ttcttggcgg ccagaaacag gtcggcgtac 4500

tggtcgccga tctgggccag caggttgtcc aggtcgtcgt cgtaggtgtc cttgctcagc 4560

tgcagtttgg catcctcggc caggtcgaag ttgctcttga agttgggggt caggcccagg 4620

ctcagggcaa tcaggtttcc gaacaggcca ttcttcttct cgccgggcag ctgggcgatc 4680

agattttcca gccgtctgct cttgctcagt ctggcagaca ggatggcctt ggcgtccacg 4740

ccgctggcgt tgatggggtt ttcctcgaac agctggttgt aggtctgcac cagctggatg 4800

aacagcttgt ccacgtcgct gttgtcgggg ttcaggtcgc cctcgatcag gaagtggccc 4860

cggaacttga tcatgtgggc cagggccaga tagatcagcc gcaggtcggc cttgtcggtg 4920

ctgtccacca gtttctttct caggtggtag atggtggggt acttctcgtg gtaggccacc 4980

tcgtccacga tgttgccgaa gatggggtgc cgctcgtgct tcttatcctc ttccaccagg 5040

aaggactctt ccagtctgtg gaagaagctg tcgtccacct tggccatctc gttgctgaag 5100

atctcttgca gatagcagat ccggttcttc cgtctggtgt atcttcttct ggcggttctc 5160

ttcagccggg tggcctcggc tgtttcgccg ctgtcgaaca gcagggctcc gatcaggttc 5220

ttcttgatgc tgtgccggtc ggtgttgccc agcaccttga atttcttgct gggcaccttg 5280

tactcgtcgg tgatcacggc ccagcccaca gagttggtgc cgatgtccag gccgatgctg 5340

tacttcttgt cggctgctgg gactccgtgg ataccgacct tccgcttctt ctttggggcc 5400

atcttatcgt catcgtcttt gtaatcaata tcatgatcct tgtagtctcc gtcgtggtcc 5460

ttatagtcca tctcgagtat cgttcgtaaa tggtgaaaat tttcagaaaa ttgcttttgc 5520

tttaaaagaa atgatttaaa ttgctgcaat agaagtagaa tgcttgattg cttgagattc 5580

gtttgttttg tatatgttgt gttgaggtcg aggtcctctc caaatgaaat gaacttcctt 5640

atatagagga agggtcttgc gaaggatagt gggattgtgc gtcatccctt acgtcagtgg 5700

agatatcaca tcaatccact tgctttgaag acgtggttgg aacgtcttct ttttccacga 5760

tgctcctcgt gggtgggggt ccatctttgg gaccactgtc ggcagaggca tcttcaacga 5820

tggcctttcc tttatcgcaa tgatggcatt tgtaggagcc accttccttt tccactatct 5880

tcacaataaa gtgacagata gctgggcaat ggaatccgag gaggtttccg gatatcaccc 5940

tttgttgaaa agtctcaatt gccctttggt cttctgagac tgtatctttg atatttttgg 6000

agtagacaag tgtgtcgtgc tccaccatgt tatcacatca atccacttgc tttgaagacg 6060

tggttggaac gtcttctttt tccacgatgc tcctcgtggg tgggggtcca tctttgggac 6120

cactgtcggc agaggcatct tcaacgatgg cctttccttt atcgcaatga tggcatttgt 6180

aggagccacc ttccttttcc actatcttca caataaagtg acagatagct gggcaatgga 6240

atccgaggag gtttccggat attacccttt gttgaaaagt ctcaattgcc ctttggtctt 6300

ctgagactgt atctttgata tttttggagt agacaagtgt gtcgtgctcc accatgttga 6360

cctgcaggca tgcaagctta tcgataccgt cgacctgcag ccaagcttat ccaacatttc 6420

acttgagtta actcaatagc aagaataacg tccatagttt cagcattcaa gcaaaacggc 6480

caagaaaatc agcttggtaa tttcagtgag acctggacta ccataagcag caccgcctat 6540

tacacttaat ggggtaaagt aaaacgagcc acatcacctc cttgatttta aggagcattt 6600

gaaggagtat aaaaagaatg tatgtaatgt aaggttgtgt tgtgtcattc aagatagcaa 6660

gacggaccaa agcttctatg tatctatcta tgtctatgat atgatgattg tattgatttg 6720

gtttgagtac agtgagggag agggaggaac ttcttcactt gtttatttaa cctgaaactc 6780

aactcaaatc actgagagtg aatgttgaga aataagtatt atgttatgtt tgctttgcta 6840

ttagtcccac atcgcttaca tatacttcag ttatattgtt tatatagcct agacgaacag 6900

cagggtttgg ctcgcaggtg aacacaacac ctgcacacgt tttagagcta gaaatagcaa 6960

gttaaaataa ggctagtccg ttatcaactt gaaaaagtgg caccgagtcg gtgctttttt 7020

tttaagcttg gcgcgccggg cccaacatgg tggagcacga cactctcgtc tactccaaga 7080

atatcaaaga tacagtctca gaagaccaaa gggctattga gacttttcaa caaagggtaa 7140

tatcgggaaa cctcctcgga ttccattgcc cagctatctg tcacttcatc aaaaggacag 7200

tagaaaagga aggtggcacc tacaaatgcc atcattgcga taaaggaaag gctatcgttc 7260

aagatgcctc tgccgacagt ggtcccaaag atggaccccc acccacgagg agcatcgtgg 7320

aaaaagaaga cgttccaacc acgtcttcaa agcaagtgga ttgatgtgaa catggtggag 7380

cacgacactc tcgtctactc caagaatatc aaagatacag tctcagaaga ccaaagggct 7440

attgagactt ttcaacaaag ggtaatatcg ggaaacctcc tcggattcca ttgcccagct 7500

atctgtcact tcatcaaaag gacagtagaa aaggaaggtg gcacctacaa atgccatcat 7560

tgcgataaag gaaaggctat cgttcaagat gcctctgccg acagtggtcc caaagatgga 7620

cccccaccca cgaggagcat cgtggaaaaa gaagacgttc caaccacgtc ttcaaagcaa 7680

gtggattgat gtgatatctc cactgacgta agggatgacg cacaatccca ctatccttcg 7740

caagaccctt cctctatata aggaagttca tttcatttgg agaggacacg ctgaaatcac 7800

cagtctctct ctacaaatct atctctctcg agctttcgca gatccggggg gcaatgagat 7860

atgaaaaagc ctgaactcac cgcgacgtct gtcgagaagt ttctgatcga aaagttcgac 7920

agcgtctccg acctgatgca gctctcggag ggcgaagaat ctcgtgcttt cagcttcgat 7980

gtaggagggc gtggatatgt cctgcgggta aatagctgcg ccgatggttt ctacaaagat 8040

cgttatgttt atcggcactt tgcatcggcc gcgctcccga ttccggaagt gcttgacatt 8100

ggggagttta gcgagagcct gacctattgc atctcccgcc gttcacaggg tgtcacgttg 8160

caagacctgc ctgaaaccga actgcccgct gttctacaac cggtcgcgga ggctatggat 8220

gcgatcgctg cggccgatct tagccagacg agcgggttcg gcccattcgg accgcaagga 8280

atcggtcaat acactacatg gcgtgatttc atatgcgcga ttgctgatcc ccatgtgtat 8340

cactggcaaa ctgtgatgga cgacaccgtc agtgcgtccg tcgcgcaggc tctcgatgag 8400

ctgatgcttt gggccgagga ctgccccgaa gtccggcacc tcgtgcacgc ggatttcggc 8460

tccaacaatg tcctgacgga caatggccgc ataacagcgg tcattgactg gagcgaggcg 8520

atgttcgggg attcccaata cgaggtcgcc aacatcttct tctggaggcc gtggttggct 8580

tgtatggagc agcagacgcg ctacttcgag cggaggcatc cggagcttgc aggatcgcca 8640

cgactccggg cgtatatgct ccgcattggt cttgaccaac tctatcagag cttggttgac 8700

ggcaatttcg atgatgcagc ttgggcgcag ggtcgatgcg acgcaatcgt ccgatccgga 8760

gccgggactg tcgggcgtac acaaatcgcc cgcagaagcg cggccgtctg gaccgatggc 8820

tgtgtagaag tactcgccga tagtggaaac cgacgcccca gcactcgtcc gagggcaaag 8880

aaatagagta gatgccgacc gggatctgtc gatcgacaag ctcgagtttc tccataataa 8940

tgtgtgagta gttcccagat aagggaatta gggttcctat agggtttcgc tcatgtgttg 9000

agcatataag aaacccttag tatgtatttg tatttgtaaa atacttctat caataaaatt 9060

tctaattcct aaaaccaaaa tccagtacta aaatccagat cccccgaatt aattcggcgt 9120

taattcagta cattaaaaac gtccgcaatg tgttattaag ttgtctaagc gtcaatttgt 9180

ttacaccaca atatatcctg ccaccagcca gccaacagct ccccgaccgg cagctcggca 9240

caaaatcacc actcgataca ggcagcccat cagtccggga cggcgtcagc gggagagccg 9300

ttgtaaggcg gcagactttg ctcatgttac cgatgctatt cggaagaacg gcaactaagc 9360

tgccgggttt gaaacacgga tgatctcgcg gagggtagca tgttgattgt aacgatgaca 9420

gagcgttgct gcctgtgatc accgcggttt caaaatcggc tccgtcgata ctatgttata 9480

cgccaacttt gaaaacaact ttgaaaaagc tgttttctgg tatttaaggt tttagaatgc 9540

aaggaacagt gaattggagt tcgtcttgtt ataattagct tcttggggta tctttaaata 9600

ctgtagaaaa gaggaaggaa ataataaatg gctaaaatga gaatatcacc ggaattgaaa 9660

aaactgatcg aaaaataccg ctgcgtaaaa gatacggaag gaatgtctcc tgctaaggta 9720

tataagctgg tgggagaaaa tgaaaaccta tatttaaaaa tgacggacag ccggtataaa 9780

gggaccacct atgatgtgga acgggaaaag gacatgatgc tatggctgga aggaaagctg 9840

cctgttccaa aggtcctgca ctttgaacgg catgatggct ggagcaatct gctcatgagt 9900

gaggccgatg gcgtcctttg ctcggaagag tatgaagatg aacaaagccc tgaaaagatt 9960

atcgagctgt atgcggagtg catcaggctc tttcactcca tcgacatatc ggattgtccc 10020

tatacgaata gcttagacag ccgcttagcc gaattggatt acttactgaa taacgatctg 10080

gccgatgtgg attgcgaaaa ctgggaagaa gacactccat ttaaagatcc gcgcgagctg 10140

tatgattttt taaagacgga aaagcccgaa gaggaacttg tcttttccca cggcgacctg 10200

ggagacagca acatctttgt gaaagatggc aaagtaagtg gctttattga tcttgggaga 10260

agcggcaggg cggacaagtg gtatgacatt gccttctgcg tccggtcgat cagggaggat 10320

atcggggaag aacagtatgt cgagctattt tttgacttac tggggatcaa gcctgattgg 10380

gagaaaataa aatattatat tttactggat gaattgtttt agtacctaga atgcatgacc 10440

aaaatccctt aacgtgagtt ttcgttccac tgagcgtcag accccgtaga aaagatcaaa 10500

ggatcttctt gagatccttt ttttctgcgc gtaatctgct gcttgcaaac aaaaaaacca 10560

ccgctaccag cggtggtttg tttgccggat caagagctac caactctttt tccgaaggta 10620

actggcttca gcagagcgca gataccaaat actgtccttc tagtgtagcc gtagttaggc 10680

caccacttca agaactctgt agcaccgcct acatacctcg ctctgctaat cctgttacca 10740

gtggctgctg ccagtggcga taagtcgtgt cttaccgggt tggactcaag acgatagtta 10800

ccggataagg cgcagcggtc gggctgaacg gggggttcgt gcacacagcc cagcttggag 10860

cgaacgacct acaccgaact gagataccta cagcgtgagc tatgagaaag cgccacgctt 10920

cccgaaggga gaaaggcgga caggtatccg gtaagcggca gggtcggaac aggagagcgc 10980

acgagggagc ttccaggggg aaacgcctgg tatctttata gtcctgtcgg gtttcgccac 11040

ctctgacttg agcgtcgatt tttgtgatgc tcgtcagggg ggcggagcct atggaaaaac 11100

gccagcaacg cggccttttt acggttcctg gccttttgct ggccttttgc tcacatgttc 11160

tttcctgcgt tatcccctga ttctgtggat aaccgtatta ccgcctttga gtgagctgat 11220

accgctcgcc gcagccgaac gaccgagcgc agcgagtcag tgagcgagga agcggaactg 11280

cgcctgatgc ggtattttct ccttacgcat ctgtgcggta tttcacaccg catatggtgc 11340

actctcagta caatctgctc tgatgccgca tagttaagcc agtatacact ccgctatcgc 11400

tacgtgactg ggtcatggct gcgccccgac acccgccaac acccgctgac gcgccctgac 11460

gggcttgtct gctcccggca tccgcttaca gacaagctgt gaccgtctcc gggagctgca 11520

tgtgtcagag gttttcaccg tcatcaccga aacgcgcgag gcagggtgcc ttgatgtggg 11580

cgccggcggt cgagtggcga cggcgcggct tgtccgcgcc ctggtagatt gcctggccgt 11640

aggccagcca tttttgagcg gccagcggcc gcgataggcc gacgcgaagc ggcggggcgt 11700

agggagcgca gcgaccgaag ggtaggcgct ttttgcagca cttcggctgt gcgctggcca 11760

gacagttatg cacaggccag gcgggtttta agagttttaa taagttttaa agagttttag 11820

gcggaaaaat cgcctttttt ctcttttata tcagtcactt acatgtgtga ccggttccca 11880

atgtacggct ttgggttccc aatgtacggg ttccggttcc caatgtacgg ctttgggttc 11940

ccaatgtacg tgctatccac aggaaagaga ccttttcgac ctttttcccc tgctagggca 12000

atttgcccta gcatctgctc cgtacattag gaaccggcgg atgcttcgcc ctcgatcagg 12060

ttgcggtagc gcatgactag gatcgggcca gcctgccccg cctcctcctt caaatcgtac 12120

tccggcaggt catttgaccc gatcagcttg cgcacggtga aacagaactt cttgaactct 12180

ccggcgctgc cactgcgttc gtagatcgtc ttgaacaacc atctggcttc tgccttgcct 12240

gcggcgcggc gtgccaggcg gtagagaaaa cggccgatgc cgggatcgat caaaaagtaa 12300

tcggggtgaa ccgtcagcac gtccgggttc ttgccttctg tgatctcgcg gtacatccaa 12360

tcagctagct cgatctcgat gtactccggc cgcccggttt cgctctttac gatcttgtag 12420

cggctaatca aggcttcacc ctcggatacc gtcaccaggc ggccgttctt ggccttcttc 12480

gtacgctgca tggcaacgtg cgtggtgttt aaccgaatgc aggtttctac caggtcgtct 12540

ttctgctttc cgccatcggc tcgccggcag aacttgagta cgtccgcaac gtgtggacgg 12600

aacacgcggc cgggcttgtc tcccttccct tcccggtatc ggttcatgga ttcggttaga 12660

tgggaaaccg ccatcagtac caggtcgtaa tcccacacac tggccatgcc ggccggccct 12720

gcggaaacct ctacgtgccc gtctggaagc tcgtagcgga tcacctcgcc agctcgtcgg 12780

tcacgcttcg acagacggaa aacggccacg tccatgatgc tgcgactatc gcgggtgccc 12840

acgtcataga gcatcggaac gaaaaaatct ggttgctcgt cgcccttggg cggcttccta 12900

atcgacggcg caccggctgc cggcggttgc cgggattctt tgcggattcg atcagcggcc 12960

gcttgccacg attcaccggg gcgtgcttct gcctcgatgc gttgccgctg ggcggcctgc 13020

gcggccttca acttctccac caggtcatca cccagcgccg cgccgatttg taccgggccg 13080

gatggtttgc gaccgctcac gccgattcct cgggcttggg ggttccagtg ccattgcagg 13140

gccggcaggc aacccagccg cttacgcctg gccaaccgcc cgttcctcca cacatggggc 13200

attccacggc gtcggtgcct ggttgttctt gattttccat gccgcctcct ttagccgcta 13260

aaattcatct actcatttat tcatttgctc atttactctg gtagctgcgc gatgtattca 13320

gatagcagct cggtaatggt cttgccttgg cgtaccgcgt acatcttcag cttggtgtga 13380

tcctccgccg gcaactgaaa gttgacccgc ttcatggctg gcgtgtctgc caggctggcc 13440

aacgttgcag ccttgctgct gcgtgcgctc ggacggccgg cacttagcgt gtttgtgctt 13500

ttgctcattt tctctttacc tcattaactc aaatgagttt tgatttaatt tcagcggcca 13560

gcgcctggac ctcgcgggca gcgtcgccct cgggttctga ttcaagaacg gttgtgccgg 13620

cggcggcagt gcctgggtag ctcacgcgct gcgtgatacg ggactcaaga atgggcagct 13680

cgtacccggc cagcgcctcg gcaacctcac cgccgatgcg cgtgcctttg atcgcccgcg 13740

acacgacaaa ggccgcttgt agccttccat ccgtgacctc aatgcgctgc ttaaccagct 13800

ccaccaggtc ggcggtggcc catatgtcgt aagggcttgg ctgcaccgga atcagcacga 13860

agtcggctgc cttgatcgcg gacacagcca agtccgccgc ctggggcgct ccgtcgatca 13920

ctacgaagtc gcgccggccg atggccttca cgtcgcggtc aatcgtcggg cggtcgatgc 13980

cgacaacggt tagcggttga tcttcccgca cggccgccca atcgcgggca ctgccctggg 14040

gatcggaatc gactaacaga acatcggccc cggcgagttg cagggcgcgg gctagatggg 14100

ttgcgatggt cgtcttgcct gacccgcctt tctggttaag tacagcgata accttcatgc 14160

gttccccttg cgtatttgtt tatttactca tcgcatcata tacgcagcga ccgcatgacg 14220

caagctgttt tactcaaata cacatcacct ttttagacgg cggcgctcgg tttcttcagc 14280

ggccaagctg gccggccagg ccgccagctt ggcatcagac aaaccggcca ggatttcatg 14340

cagccgcacg gttgagacgt gcgcgggcgg ctcgaacacg tacccggccg cgatcatctc 14400

cgcctcgatc tcttcggtaa tgaaaaacgg ttcgtcctgg ccgtcctggt gcggtttcat 14460

gcttgttcct cttggcgttc attctcggcg gccgccaggg cgtcggcctc ggtcaatgcg 14520

tcctcacgga aggcaccgcg ccgcctggcc tcggtgggcg tcacttcctc gctgcgctca 14580

agtgcgcggt acagggtcga gcgatgcacg ccaagcagtg cagccgcctc tttcacggtg 14640

cggccttcct ggtcgatcag ctcgcgggcg tgcgcgatct gtgccggggt gagggtaggg 14700

cgggggccaa acttcacgcc tcgggccttg gcggcctcgc gcccgctccg ggtgcggtcg 14760

atgattaggg aacgctcgaa ctcggcaatg ccggcgaaca cggtcaacac catgcggccg 14820

gccggcgtgg tggtgtcggc ccacggctct gccaggctac gcaggcccgc gccggcctcc 14880

tggatgcgct cggcaatgtc cagtaggtcg cgggtgctgc gggccaggcg gtctagcctg 14940

gtcactgtca c 14951

<210> 2

<211> 500

<212> DNA

<213> artificial sequence

<220>

<223> MtU6 promoter

<400> 2

atccaacatt tcacttgagt taactcaata gcaagaataa cgtccatagt ttcagcattc 60

aagcaaaacg gccaagaaaa tcagcttggt aatttcagtg agacctggac taccataagc 120

agcaccgcct attacactta atggggtaaa gtaaaacgag ccacatcacc tccttgattt 180

taaggagcat ttgaaggagt ataaaaagaa tgtatgtaat gtaaggttgt gttgtgtcat 240

tcaagatagc aagacggacc aaagcttcta tgtatctatc tatgtctatg atatgatgat 300

tgtattgatt tggtttgagt acagtgaggg agagggagga acttcttcac ttgtttattt 360

aacctgaaac tcaactcaaa tcactgagag tgaatgttga gaaataagta ttatgttatg 420

tttgctttgc tattagtccc acatcgctta catatacttc agttatattg tttatatagc 480

ctagacgaac agcagggttt 500

<210> 3

<211> 115

<212> DNA

<213> artificial sequence

<220>

<223> DNA sequence expressing sgRNA

<400> 3

ggctcgcagg tgaacacaac acctgcacac gttttagagc tagaaatagc aagttaaaat 60

aaggctagtc cgttatcaac ttgaaaaagt ggcaccgagt cggtgctttt ttttt 115

<210> 4

<211> 21

<212> DNA

<213> artificial sequence

<220>

<223> sequencing primer MtU6-T-F

<400> 4

ggcatgcaag cttatcgata c 21

SEQUENCE LISTING

<110>  GUANGDONG SANJIE FORAGE BIOTECHNOLOGY CO., LTD

<120>  Method for site-specific mutagenesis of Medicago sativa gene by
       using CRISPR/Cas9 system

<130>  GWPCTP202011375

<160>  4

<170>  PatentIn version 3.5

<210>  1
<211>  14951
<212>  DNA
<213>  artificial sequence

<220>
<223>  binary expression vector MsCRISPR/Cas9

<400>  1
aacgtcgcca gggcgtaggt ggtcaagcat cctggccagc tccgggcggt cgcgcctggt      60

gccggtgatc ttctcggaaa acagcttggt gcagccggcc gcgtgcagtt cggcccgttg     120

gttggtcaag tcctggtcgt cggtgctgac gcgggcatag cccagcaggc cagcggcggc     180

gctcttgttc atggcgtaat gtctccggtt ctagtcgcaa gtattctact ttatgcgact     240

aaaacacgcg acaagaaaac gccaggaaaa gggcagggcg gcagcctgtc gcgtaactta     300

ggacttgtgc gacatgtcgt tttcagaaga cggctgcact gaacgtcaga agccgactgc     360

actatagcag cggaggggtt ggatcaaagt actttgatcc cgaggggaac cctgtggttg     420

gcatgcacat acaaatggac gaacggataa accttttcac gcccttttaa atatccgatt     480

attctaataa acgctctttt ctcttaggtt tacccgccaa tatatcctgt caaacactga     540

tagtttaatt cccgatctag taacatagat gacaccgcgc gcgataattt atcctagttt     600

gcgcgctata ttttgttttc tatcgcgtat taaatgtata attgcgggac tctaatcata     660

aaaacccatc tcataaataa cgtcatgcat tacatgttaa ttattacatg cttaacgtaa     720

ttcaacagaa attatatgat aatcatcgca agaccggcaa caggattcaa tcttaagaaa     780

ctttattgcc aaatgtttga acgatcgggg aaattcgagc tggtcacctg taattcacac     840

gtggtggtgg tggtggtggc tagcgttaac actagtcaga tctaccatgg tggactcctc     900

ttagaattcc cgatctagta acatagatga caccgcgcgc gataatttat cctagtttgc     960

gcgctatatt ttgttttcta tcgcgtatta aatgtataat tgcgggactc taatcataaa    1020

aacccatctc ataaataacg tcatgcatta catgttaatt attacatgct taacgtaatt    1080

caacagaaat tatatgataa tcatcgcaag accggcaaca ggattcaatc ttaagaaact    1140

ttattgccaa atgtttgaac gatcggggaa attcgagctc tatcgatcaa tcaggatcct    1200

tactttttct tttttgcctg gccggccttt ttcgtggccg ccggcctttt gtcgcctccc    1260

agctgagaca ggtcgatccg tgtctcgtac aggccggtga tgctctggtg gatcagggtg 1320

gcgtccagca cctctttggt gctggtgtac ctcttccggt cgatggtggt gtcaaagtac 1380

ttgaaggcgg caggggctcc cagattggtc agggtaaaca ggtggatgat attctcggcc 1440

tgctctctga tgggcttatc ccggtgcttg ttgtaggcgg acagcacttt gtccagatta 1500

gcgtcggcca ggatcactct cttggagaac tcgctgatct gctcgatgat ctcgtccagg 1560

tagtgcttgt gctgttccac aaacagctgt ttctgctcat tatcctcggg ggagcccttc 1620

agcttctcat agtggctggc caggtacagg aagttcacat atttggaggg cagggccagt 1680

tcgtttccct tctgcagttc gccggcagag gccagcattc tcttccggcc gttttccagc 1740

tcgaacaggg agtacttagg cagcttgatg atcaggtcct ttttcacttc tttgtagccc 1800

ttggcttcca gaaagtcgat gggattcttc tcgaagctgc ttctttccat gatggtgatc 1860

cccagcagct ctttcacact cttcagtttc ttggacttgc ccttttccac tttggccacc 1920

accagcacag aataggccac ggtggggctg tcgaagccgc cgtacttctt agggtcccag 1980

tccttctttc tggcgatcag cttatcgctg ttcctcttgg gcaggataga ctctttgctg 2040

aagccgcctg tctgcacctc ggtctttttc acgatattca cttggggcat gctcagcact 2100

ttccgcacgg tggcaaaatc ccggccctta tcccacacga tctccccggt ttcgccgttt 2160

gtctcgatca gaggccgctt ccggatctcg ccgttggcca gggtaatctc ggtcttgaaa 2220

aagttcatga tgttgctgta gaagaagtac ttggcggtag ccttgccgat ttcctgctcg 2280

ctcttggcga tcatcttccg cacgtcgtac accttgtagt cgccgtacac gaactcgctt 2340

tccagcttag ggtacttttt gatcagggcg gttcccacga cggcgttcag gtaggcgtcg 2400

tgggcgtggt ggtagttgtt gatctcgcgc actttgtaaa actggaaatc cttccggaaa 2460

tcggacacca gcttggactt cagggtgatc actttcactt cccggatcag cttgtcattc 2520

tcgtcgtact tagtgttcat ccgggagtcc aggatctgtg ccacgtgctt tgtgatctgc 2580

cgggtttcca ccagctgtct cttgatgaag ccggccttat ccagttcgct caggccgcct 2640

ctctcggcct tggtcagatt gtcgaacttt ctctgggtaa tcagcttggc gttcagcagc 2700

tgccgccagt agttcttcat cttcttcacg acctcttcgg agggcacgtt gtcgctcttg 2760

ccccggttct tgtcgcttct ggtcagcacc ttgttgtcga tggagtcgtc cttcagaaag 2820

ctctgaggca cgatatggtc cacatcgtag tcggacagcc ggttgatgtc cagttcctgg 2880

tccacgtaca tatcccgccc attctgcagg tagtacaggt acagcttctc gttctgcagc 2940

tgggtgtttt ccacggggtg ttctttcagg atctggctgc ccagctcttt gatgccctct 3000

tcgatccgct tcattctctc gcggctgttc ttctgtccct tctgggtggt ctggttctct 3060

ctggccattt cgatcacgat gttctcgggc ttgtgccggc ccatcacttt cacgagctcg 3120

tccaccacct tcactgtctg caggatgccc ttcttaatgg cggggctgcc ggccagattg 3180

gcaatgtgct cgtgcaggct atcgccctgg ccggacacct gggctttctg gatgtcctct 3240

ttaaaggtca ggctgtcgtc gtggatcagc tgcatgaagt ttctgttggc gaagccgtcg 3300

gacttcagga aatccaggat tgtcttgccg gactgcttgt cccggatgcc gttgatcagc 3360

ttccggctca gcctgcccca gccggtgtat ctccgccgct tcagctgctt catcactttg 3420

tcgtcgaaca ggtgggcata ggttttcagc cgttcctcga tcatctctct gtcctcaaac 3480

agtgtcaggg tcagcacgat atcttccaga atgtcctcgt tttcctcatt gtccaggaag 3540

tccttgtcct tgataatttt cagcagatcg tggtatgtgc ccagggaggc gttgaaccga 3600

tcttccacgc cggagatttc cacggagtcg aagcactcga ttttcttgaa gtagtcctct 3660

ttcagctgct tcacggtcac tttccggttg gtcttgaaca gcaggtccac gatggccttt 3720

ttctgctcgc cgctcaggaa ggcgggcttt ctcattccct cggtcacgta tttcactttg 3780

gtcagctcgt tatacacggt gaagtactcg tacagcaggc tgtgcttggg cagcaccttc 3840

tcgttgggca ggttcttatc gaagttggtc atccgctcga tgaagctctg ggcggaagcg 3900

cccttgtcca ccacttcctc gaagttccag ggggtgatgg tttcctcgct ctttctggtc 3960

atccaggcga atctgctgtt tcccctggcc agagggccca cgtagtaggg gatgcggaag 4020

gtcaggatct tctcgatctt ttcccggttg tccttcagga atgggtaaaa atcttcctgc 4080

cgccgcagaa tggcgtgcag ctctcccagg tggatctggt gggggatgct gccgttgtcg 4140

aaggtccgct gcttccgcag caggtcctct ctgttcagct tcacgagcag ttcctcggtg 4200

ccgtccatct tttccaggat gggcttgatg aacttgtaga actcttcctg gctggctccg 4260

ccgtcaatgt agccggcgta gccgttcttg ctctggtcga agaaaatctc tttgtacttc 4320

tcaggcagct gctgccgcac gagagctttc agcagggtca ggtcctggtg gtgctcgtcg 4380

tatctcttga tcatagaggc gctcaggggg gccttggtga tctcggtgtt cactctcagg 4440

atgtcgctca gcaggatggc gtcggacagg ttcttggcgg ccagaaacag gtcggcgtac 4500

tggtcgccga tctgggccag caggttgtcc aggtcgtcgt cgtaggtgtc cttgctcagc 4560

tgcagtttgg catcctcggc caggtcgaag ttgctcttga agttgggggt caggcccagg 4620

ctcagggcaa tcaggtttcc gaacaggcca ttcttcttct cgccgggcag ctgggcgatc 4680

agattttcca gccgtctgct cttgctcagt ctggcagaca ggatggcctt ggcgtccacg 4740

ccgctggcgt tgatggggtt ttcctcgaac agctggttgt aggtctgcac cagctggatg 4800

aacagcttgt ccacgtcgct gttgtcgggg ttcaggtcgc cctcgatcag gaagtggccc 4860

cggaacttga tcatgtgggc cagggccaga tagatcagcc gcaggtcggc cttgtcggtg 4920

ctgtccacca gtttctttct caggtggtag atggtggggt acttctcgtg gtaggccacc 4980

tcgtccacga tgttgccgaa gatggggtgc cgctcgtgct tcttatcctc ttccaccagg 5040

aaggactctt ccagtctgtg gaagaagctg tcgtccacct tggccatctc gttgctgaag 5100

atctcttgca gatagcagat ccggttcttc cgtctggtgt atcttcttct ggcggttctc 5160

ttcagccggg tggcctcggc tgtttcgccg ctgtcgaaca gcagggctcc gatcaggttc 5220

ttcttgatgc tgtgccggtc ggtgttgccc agcaccttga atttcttgct gggcaccttg 5280

tactcgtcgg tgatcacggc ccagcccaca gagttggtgc cgatgtccag gccgatgctg 5340

tacttcttgt cggctgctgg gactccgtgg ataccgacct tccgcttctt ctttggggcc 5400

atcttatcgt catcgtcttt gtaatcaata tcatgatcct tgtagtctcc gtcgtggtcc 5460

ttatagtcca tctcgagtat cgttcgtaaa tggtgaaaat tttcagaaaa ttgcttttgc 5520

tttaaaagaa atgatttaaa ttgctgcaat agaagtagaa tgcttgattg cttgagattc 5580

gtttgttttg tatatgttgt gttgaggtcg aggtcctctc caaatgaaat gaacttcctt 5640

atatagagga agggtcttgc gaaggatagt gggattgtgc gtcatccctt acgtcagtgg 5700

agatatcaca tcaatccact tgctttgaag acgtggttgg aacgtcttct ttttccacga 5760

tgctcctcgt gggtgggggt ccatctttgg gaccactgtc ggcagaggca tcttcaacga 5820

tggcctttcc tttatcgcaa tgatggcatt tgtaggagcc accttccttt tccactatct 5880

tcacaataaa gtgacagata gctgggcaat ggaatccgag gaggtttccg gatatcaccc 5940

tttgttgaaa agtctcaatt gccctttggt cttctgagac tgtatctttg atatttttgg 6000

agtagacaag tgtgtcgtgc tccaccatgt tatcacatca atccacttgc tttgaagacg 6060

tggttggaac gtcttctttt tccacgatgc tcctcgtggg tgggggtcca tctttgggac 6120

cactgtcggc agaggcatct tcaacgatgg cctttccttt atcgcaatga tggcatttgt 6180

aggagccacc ttccttttcc actatcttca caataaagtg acagatagct gggcaatgga 6240

atccgaggag gtttccggat attacccttt gttgaaaagt ctcaattgcc ctttggtctt 6300

ctgagactgt atctttgata tttttggagt agacaagtgt gtcgtgctcc accatgttga 6360

cctgcaggca tgcaagctta tcgataccgt cgacctgcag ccaagcttat ccaacatttc 6420

acttgagtta actcaatagc aagaataacg tccatagttt cagcattcaa gcaaaacggc 6480

caagaaaatc agcttggtaa tttcagtgag acctggacta ccataagcag caccgcctat 6540

tacacttaat ggggtaaagt aaaacgagcc acatcacctc cttgatttta aggagcattt 6600

gaaggagtat aaaaagaatg tatgtaatgt aaggttgtgt tgtgtcattc aagatagcaa 6660

gacggaccaa agcttctatg tatctatcta tgtctatgat atgatgattg tattgatttg 6720

gtttgagtac agtgagggag agggaggaac ttcttcactt gtttatttaa cctgaaactc 6780

aactcaaatc actgagagtg aatgttgaga aataagtatt atgttatgtt tgctttgcta 6840

ttagtcccac atcgcttaca tatacttcag ttatattgtt tatatagcct agacgaacag 6900

cagggtttgg ctcgcaggtg aacacaacac ctgcacacgt tttagagcta gaaatagcaa 6960

gttaaaataa ggctagtccg ttatcaactt gaaaaagtgg caccgagtcg gtgctttttt 7020

tttaagcttg gcgcgccggg cccaacatgg tggagcacga cactctcgtc tactccaaga 7080

atatcaaaga tacagtctca gaagaccaaa gggctattga gacttttcaa caaagggtaa 7140

tatcgggaaa cctcctcgga ttccattgcc cagctatctg tcacttcatc aaaaggacag 7200

tagaaaagga aggtggcacc tacaaatgcc atcattgcga taaaggaaag gctatcgttc 7260

aagatgcctc tgccgacagt ggtcccaaag atggaccccc acccacgagg agcatcgtgg 7320

aaaaagaaga cgttccaacc acgtcttcaa agcaagtgga ttgatgtgaa catggtggag 7380

cacgacactc tcgtctactc caagaatatc aaagatacag tctcagaaga ccaaagggct 7440

attgagactt ttcaacaaag ggtaatatcg ggaaacctcc tcggattcca ttgcccagct 7500

atctgtcact tcatcaaaag gacagtagaa aaggaaggtg gcacctacaa atgccatcat 7560

tgcgataaag gaaaggctat cgttcaagat gcctctgccg acagtggtcc caaagatgga 7620

cccccaccca cgaggagcat cgtggaaaaa gaagacgttc caaccacgtc ttcaaagcaa 7680

gtggattgat gtgatatctc cactgacgta agggatgacg cacaatccca ctatccttcg 7740

caagaccctt cctctatata aggaagttca tttcatttgg agaggacacg ctgaaatcac 7800

cagtctctct ctacaaatct atctctctcg agctttcgca gatccggggg gcaatgagat 7860

atgaaaaagc ctgaactcac cgcgacgtct gtcgagaagt ttctgatcga aaagttcgac 7920

agcgtctccg acctgatgca gctctcggag ggcgaagaat ctcgtgcttt cagcttcgat 7980

gtaggagggc gtggatatgt cctgcgggta aatagctgcg ccgatggttt ctacaaagat 8040

cgttatgttt atcggcactt tgcatcggcc gcgctcccga ttccggaagt gcttgacatt 8100

ggggagttta gcgagagcct gacctattgc atctcccgcc gttcacaggg tgtcacgttg 8160

caagacctgc ctgaaaccga actgcccgct gttctacaac cggtcgcgga ggctatggat 8220

gcgatcgctg cggccgatct tagccagacg agcgggttcg gcccattcgg accgcaagga 8280

atcggtcaat acactacatg gcgtgatttc atatgcgcga ttgctgatcc ccatgtgtat 8340

cactggcaaa ctgtgatgga cgacaccgtc agtgcgtccg tcgcgcaggc tctcgatgag 8400

ctgatgcttt gggccgagga ctgccccgaa gtccggcacc tcgtgcacgc ggatttcggc 8460

tccaacaatg tcctgacgga caatggccgc ataacagcgg tcattgactg gagcgaggcg 8520

atgttcgggg attcccaata cgaggtcgcc aacatcttct tctggaggcc gtggttggct 8580

tgtatggagc agcagacgcg ctacttcgag cggaggcatc cggagcttgc aggatcgcca 8640

cgactccggg cgtatatgct ccgcattggt cttgaccaac tctatcagag cttggttgac 8700

ggcaatttcg atgatgcagc ttgggcgcag ggtcgatgcg acgcaatcgt ccgatccgga 8760

gccgggactg tcgggcgtac acaaatcgcc cgcagaagcg cggccgtctg gaccgatggc 8820

tgtgtagaag tactcgccga tagtggaaac cgacgcccca gcactcgtcc gagggcaaag 8880

aaatagagta gatgccgacc gggatctgtc gatcgacaag ctcgagtttc tccataataa 8940

tgtgtgagta gttcccagat aagggaatta gggttcctat agggtttcgc tcatgtgttg 9000

agcatataag aaacccttag tatgtatttg tatttgtaaa atacttctat caataaaatt 9060

tctaattcct aaaaccaaaa tccagtacta aaatccagat cccccgaatt aattcggcgt 9120

taattcagta cattaaaaac gtccgcaatg tgttattaag ttgtctaagc gtcaatttgt 9180

ttacaccaca atatatcctg ccaccagcca gccaacagct ccccgaccgg cagctcggca 9240

caaaatcacc actcgataca ggcagcccat cagtccggga cggcgtcagc gggagagccg 9300

ttgtaaggcg gcagactttg ctcatgttac cgatgctatt cggaagaacg gcaactaagc 9360

tgccgggttt gaaacacgga tgatctcgcg gagggtagca tgttgattgt aacgatgaca 9420

gagcgttgct gcctgtgatc accgcggttt caaaatcggc tccgtcgata ctatgttata 9480

cgccaacttt gaaaacaact ttgaaaaagc tgttttctgg tatttaaggt tttagaatgc 9540

aaggaacagt gaattggagt tcgtcttgtt ataattagct tcttggggta tctttaaata 9600

ctgtagaaaa gaggaaggaa ataataaatg gctaaaatga gaatatcacc ggaattgaaa 9660

aaactgatcg aaaaataccg ctgcgtaaaa gatacggaag gaatgtctcc tgctaaggta 9720

tataagctgg tgggagaaaa tgaaaaccta tatttaaaaa tgacggacag ccggtataaa 9780

gggaccacct atgatgtgga acgggaaaag gacatgatgc tatggctgga aggaaagctg 9840

cctgttccaa aggtcctgca ctttgaacgg catgatggct ggagcaatct gctcatgagt 9900

gaggccgatg gcgtcctttg ctcggaagag tatgaagatg aacaaagccc tgaaaagatt 9960

atcgagctgt atgcggagtg catcaggctc tttcactcca tcgacatatc ggattgtccc 10020

tatacgaata gcttagacag ccgcttagcc gaattggatt acttactgaa taacgatctg 10080

gccgatgtgg attgcgaaaa ctgggaagaa gacactccat ttaaagatcc gcgcgagctg 10140

tatgattttt taaagacgga aaagcccgaa gaggaacttg tcttttccca cggcgacctg 10200

ggagacagca acatctttgt gaaagatggc aaagtaagtg gctttattga tcttgggaga 10260

agcggcaggg cggacaagtg gtatgacatt gccttctgcg tccggtcgat cagggaggat 10320

atcggggaag aacagtatgt cgagctattt tttgacttac tggggatcaa gcctgattgg 10380

gagaaaataa aatattatat tttactggat gaattgtttt agtacctaga atgcatgacc 10440

aaaatccctt aacgtgagtt ttcgttccac tgagcgtcag accccgtaga aaagatcaaa 10500

ggatcttctt gagatccttt ttttctgcgc gtaatctgct gcttgcaaac aaaaaaacca 10560

ccgctaccag cggtggtttg tttgccggat caagagctac caactctttt tccgaaggta 10620

actggcttca gcagagcgca gataccaaat actgtccttc tagtgtagcc gtagttaggc 10680

caccacttca agaactctgt agcaccgcct acatacctcg ctctgctaat cctgttacca 10740

gtggctgctg ccagtggcga taagtcgtgt cttaccgggt tggactcaag acgatagtta 10800

ccggataagg cgcagcggtc gggctgaacg gggggttcgt gcacacagcc cagcttggag 10860

cgaacgacct acaccgaact gagataccta cagcgtgagc tatgagaaag cgccacgctt 10920

cccgaaggga gaaaggcgga caggtatccg gtaagcggca gggtcggaac aggagagcgc 10980

acgagggagc ttccaggggg aaacgcctgg tatctttata gtcctgtcgg gtttcgccac 11040

ctctgacttg agcgtcgatt tttgtgatgc tcgtcagggg ggcggagcct atggaaaaac 11100

gccagcaacg cggccttttt acggttcctg gccttttgct ggccttttgc tcacatgttc 11160

tttcctgcgt tatcccctga ttctgtggat aaccgtatta ccgcctttga gtgagctgat 11220

accgctcgcc gcagccgaac gaccgagcgc agcgagtcag tgagcgagga agcggaactg 11280

cgcctgatgc ggtattttct ccttacgcat ctgtgcggta tttcacaccg catatggtgc 11340

actctcagta caatctgctc tgatgccgca tagttaagcc agtatacact ccgctatcgc 11400

tacgtgactg ggtcatggct gcgccccgac acccgccaac acccgctgac gcgccctgac 11460

gggcttgtct gctcccggca tccgcttaca gacaagctgt gaccgtctcc gggagctgca 11520

tgtgtcagag gttttcaccg tcatcaccga aacgcgcgag gcagggtgcc ttgatgtggg 11580

cgccggcggt cgagtggcga cggcgcggct tgtccgcgcc ctggtagatt gcctggccgt 11640

aggccagcca tttttgagcg gccagcggcc gcgataggcc gacgcgaagc ggcggggcgt 11700

agggagcgca gcgaccgaag ggtaggcgct ttttgcagca cttcggctgt gcgctggcca 11760

gacagttatg cacaggccag gcgggtttta agagttttaa taagttttaa agagttttag 11820

gcggaaaaat cgcctttttt ctcttttata tcagtcactt acatgtgtga ccggttccca 11880

atgtacggct ttgggttccc aatgtacggg ttccggttcc caatgtacgg ctttgggttc 11940

ccaatgtacg tgctatccac aggaaagaga ccttttcgac ctttttcccc tgctagggca 12000

atttgcccta gcatctgctc cgtacattag gaaccggcgg atgcttcgcc ctcgatcagg 12060

ttgcggtagc gcatgactag gatcgggcca gcctgccccg cctcctcctt caaatcgtac 12120

tccggcaggt catttgaccc gatcagcttg cgcacggtga aacagaactt cttgaactct 12180

ccggcgctgc cactgcgttc gtagatcgtc ttgaacaacc atctggcttc tgccttgcct 12240

gcggcgcggc gtgccaggcg gtagagaaaa cggccgatgc cgggatcgat caaaaagtaa 12300

tcggggtgaa ccgtcagcac gtccgggttc ttgccttctg tgatctcgcg gtacatccaa 12360

tcagctagct cgatctcgat gtactccggc cgcccggttt cgctctttac gatcttgtag 12420

cggctaatca aggcttcacc ctcggatacc gtcaccaggc ggccgttctt ggccttcttc 12480

gtacgctgca tggcaacgtg cgtggtgttt aaccgaatgc aggtttctac caggtcgtct 12540

ttctgctttc cgccatcggc tcgccggcag aacttgagta cgtccgcaac gtgtggacgg 12600

aacacgcggc cgggcttgtc tcccttccct tcccggtatc ggttcatgga ttcggttaga 12660

tgggaaaccg ccatcagtac caggtcgtaa tcccacacac tggccatgcc ggccggccct 12720

gcggaaacct ctacgtgccc gtctggaagc tcgtagcgga tcacctcgcc agctcgtcgg 12780

tcacgcttcg acagacggaa aacggccacg tccatgatgc tgcgactatc gcgggtgccc 12840

acgtcataga gcatcggaac gaaaaaatct ggttgctcgt cgcccttggg cggcttccta 12900

atcgacggcg caccggctgc cggcggttgc cgggattctt tgcggattcg atcagcggcc 12960

gcttgccacg attcaccggg gcgtgcttct gcctcgatgc gttgccgctg ggcggcctgc 13020

gcggccttca acttctccac caggtcatca cccagcgccg cgccgatttg taccgggccg 13080

gatggtttgc gaccgctcac gccgattcct cgggcttggg ggttccagtg ccattgcagg 13140

gccggcaggc aacccagccg cttacgcctg gccaaccgcc cgttcctcca cacatggggc 13200

attccacggc gtcggtgcct ggttgttctt gattttccat gccgcctcct ttagccgcta 13260

aaattcatct actcatttat tcatttgctc atttactctg gtagctgcgc gatgtattca 13320

gatagcagct cggtaatggt cttgccttgg cgtaccgcgt acatcttcag cttggtgtga 13380

tcctccgccg gcaactgaaa gttgacccgc ttcatggctg gcgtgtctgc caggctggcc 13440

aacgttgcag ccttgctgct gcgtgcgctc ggacggccgg cacttagcgt gtttgtgctt 13500

ttgctcattt tctctttacc tcattaactc aaatgagttt tgatttaatt tcagcggcca 13560

gcgcctggac ctcgcgggca gcgtcgccct cgggttctga ttcaagaacg gttgtgccgg 13620

cggcggcagt gcctgggtag ctcacgcgct gcgtgatacg ggactcaaga atgggcagct 13680

cgtacccggc cagcgcctcg gcaacctcac cgccgatgcg cgtgcctttg atcgcccgcg 13740

acacgacaaa ggccgcttgt agccttccat ccgtgacctc aatgcgctgc ttaaccagct 13800

ccaccaggtc ggcggtggcc catatgtcgt aagggcttgg ctgcaccgga atcagcacga 13860

agtcggctgc cttgatcgcg gacacagcca agtccgccgc ctggggcgct ccgtcgatca 13920

ctacgaagtc gcgccggccg atggccttca cgtcgcggtc aatcgtcggg cggtcgatgc 13980

cgacaacggt tagcggttga tcttcccgca cggccgccca atcgcgggca ctgccctggg 14040

gatcggaatc gactaacaga acatcggccc cggcgagttg cagggcgcgg gctagatggg 14100

ttgcgatggt cgtcttgcct gacccgcctt tctggttaag tacagcgata accttcatgc 14160

gttccccttg cgtatttgtt tatttactca tcgcatcata tacgcagcga ccgcatgacg 14220

caagctgttt tactcaaata cacatcacct ttttagacgg cggcgctcgg tttcttcagc 14280

ggccaagctg gccggccagg ccgccagctt ggcatcagac aaaccggcca ggatttcatg 14340

cagccgcacg gttgagacgt gcgcgggcgg ctcgaacacg tacccggccg cgatcatctc 14400

cgcctcgatc tcttcggtaa tgaaaaacgg ttcgtcctgg ccgtcctggt gcggtttcat 14460

gcttgttcct cttggcgttc attctcggcg gccgccaggg cgtcggcctc ggtcaatgcg 14520

tcctcacgga aggcaccgcg ccgcctggcc tcggtgggcg tcacttcctc gctgcgctca 14580

agtgcgcggt acagggtcga gcgatgcacg ccaagcagtg cagccgcctc tttcacggtg 14640

cggccttcct ggtcgatcag ctcgcgggcg tgcgcgatct gtgccggggt gagggtaggg 14700

cgggggccaa acttcacgcc tcgggccttg gcggcctcgc gcccgctccg ggtgcggtcg 14760

atgattaggg aacgctcgaa ctcggcaatg ccggcgaaca cggtcaacac catgcggccg 14820

gccggcgtgg tggtgtcggc ccacggctct gccaggctac gcaggcccgc gccggcctcc 14880

tggatgcgct cggcaatgtc cagtaggtcg cgggtgctgc gggccaggcg gtctagcctg 14940

gtcactgtca c 14951

<210> 2
<211> 500
<212> DNA
<213> artificial sequence

<220>
<223> MtU6 promoter

<400> 2
atccaacatt tcacttgagt taactcaata gcaagaataa cgtccatagt ttcagcattc 60

aagcaaaacg gccaagaaaa tcagcttggt aatttcagtg agacctggac taccataagc 120

agcaccgcct attacactta atggggtaaa gtaaaacgag ccacatcacc tccttgattt 180

taaggagcat ttgaaggagt ataaaaagaa tgtatgtaat gtaaggttgt gttgtgtcat 240

tcaagatagc aagacggacc aaagcttcta tgtatctatc tatgtctatg atatgatgat 300

tgtattgatt tggtttgagt acagtgaggg agagggagga acttcttcac ttgtttattt 360

aacctgaaac tcaactcaaa tcactgagag tgaatgttga gaaataagta ttatgttatg 420

tttgctttgc tattagtccc acatcgctta catatacttc agttatattg tttatatagc 480

ctagacgaac agcagggttt 500

<210> 3
<211> 115
<212> DNA
<213> artificial sequence

<220>
<223> DNA sequence expressing sgRNA

<400> 3
ggctcgcagg tgaacacaac acctgcacac gttttagagc tagaaatagc aagttaaaat 60

aaggctagtc cgttatcaac ttgaaaaagt ggcaccgagt cggtgctttt ttttt 115

<210> 4
<211> 21

<212>  DNA
<213>  artificial sequence

<220>
<223>  sequencing primer MtU6-T-F

<400>  4
ggcatgcaag cttatcgata c                                                21

## Description

### TECHNICAL FIELD

The present disclosure belongs to the technical field of genome editing, and specifically pertains to a method for site-specific mutagenesis of *Medicago sativa* genes by using a CRISPR/Cas9 system.

### BACKGROUD

*Medicago sativa* is a kind of legume forage cultivated widely, being rich in high quality dietary fibres, edible proteins, various vitamins (including vitamin B, vitamin C, vitamin E, etc.), various beneficial minerals as well as saponin, flavonoids, carotenoids, phenolic acid and other bioactive components, high in protein content and having good palatability, which is of great significance for improving the feed level of livestocks. In addition to being forage crops, the well-developed root system of *Medicago sativa* plays an important role in preventing and controlling soil erosion as well as environmental management. In addition, because the roots of *Medicago sativa* are symbiotic with a large number of nitrogen-fixing microorganisms, so *Medicago sativa* also has important significance in improving the fertility of soil. Researches on gene functions of *Medicago sativa* are helpful to improve the agronomic traits of *Medicago sativa.* Using gene silencing technologies to suppress the expression of target genes in *Medicago sativa* is a very efficient means to study gene functions and cultivate new varieties.

Although spontaneous mutations often occur in nature, their frequency is extremely low. The mutation rate of spontaneous mutations at most loci in each generation is about 10⁻⁵ or 10⁻⁶, and only mutations that occur in gametes can be passed on to the next generation. Ideal mutants can be created in a short time through treating plant materials by chemical mutagenesis, physical mutagenesis as well as DNA insertional mutagenesis. The frequency of mutations caused by mutagens is high, which is hundreds or even thousands of times higher than the frequency of spontaneous mutations. Moreover, mutagens-induced mutations have a wide range of variations and various types, sometimes may induce new mutations that are rare or have not occurred in nature. However, mutations introduced by mutagens are also random, and it is very difficult to generate null mutants for a specific site. Similarly, mutations induced by mutagenesis can be passed on to the next generation only if they occur in gametes. Targeted gene silencing is an effective method to study specific gene functions. At present, several genome editing techniques such as RNAi (RNA interference), as well as ZFN, TALLEN, CRISPR/Cas9 have been developed for targeted gene silencing. The RNAi technology mainly degrades mRNA sequences of the target genes and down-regulate the translation level of mRNA, thus inhibiting the expression of target genes. However, when the expression of target genes is reduced by the RNAi technology, the gene sequence of the target genes has not been mutated. In contrast, gene silencing by means of ZFN, TALLEN, CRISPR/Cas9 technologies makes the gene sequence mutated at the DNA level, resulting in complete loss of gene function. Compared with RNAi, the three technologies are more stable and efficient. When using TALEN and ZFN technologies to silence the target genes, the amino acid sequence elements that recognize different bases are assembled according to the target site, wherein each target site has its own unique protein sequence, thus resulting in a time-consuming, labor-intensive and costly process. Different from TALLEN and ZFN, the CRISPR/Cas9 technology identify the target site through the guide sequence on sgRNA. The guide sequence recognizes the target site by complementary base pairing, which is more convenient, cheap and easy to operate. Therefore, since the CRISPR/Cas9 technology was firstly applied in genome editing in 2013, it has developed rapidly and extensively. The CRISPR/Cas9 system is an acquired immune system of *Streptococcus,* which can resist the invasion of foreign genes. It is composed of CRISPR and specific Cas9 protein.CRISPR (Clustered Regularly Interspaced Short Palindromic Repeats) is a new type of DNA repeating sequence, which consists of a series of short highly conserved forward repeating sequences and spaced sequences arranged alternately. Cas9 protein is a kind of multidomain protein consisting of 1,409 amino acids, which contains two nuclease domains, RuvC-like and HNH. The CRISPR/Cas9-based genome editing system includes 2 parts: sgRNA and enzyme Cas9. Such a system can specifically identify the target site adjacent to PAM (NGG) motif, and cut it at 3nt upstream of PAM to form a DSB (double strains break), after then the organism utilizes its own repairing mechanism, such as homologous recombination (HR) and non-homologous end joining (NHEJ), to repair the damaged DNA. The repairing process of NHEJ can produce Indel (including deletions and insertions), which further leads to frameshift mutation of the DNA coding sequence.

*Medicago sativa* is a strictly cross-pollinated autotetraploid plant, with four alleles at each locus. For a particular locus, the most ideal way to generate null mutant strains with stable inheritance is creating enough mutants with four alleles simultaneously mutated. Null mutant strains can also be generated by repeatedly hybridizing the natural or mutagenic induced heterozygous mutants to accumulate the mutations, but this is a time-consuming, labor-intensive, high input, and inefficient process. Therefore, in conclusion, cultivating novel alfalfa varieties by recurrent hybridization and selection is particularly difficult, especially for recessive gene mutations. It is of great significance for the researching and breeding of *Medicago sativa* to develop an efficient targeted mutation technology that can be used to create enough null mutants within one generation. Currently, it is known to employ the RNAi technology to reduce the gene expression level of *Medicago sativa,* and there have been no reports of mutants created by using genome editing ZFN, TALEN, and CRISPR/Cas9. As described above, compared with the genome editing technologies such as ZFN, TALEN, and CRISPR/Cas9, RNAi restricts its own application due to incomplete silencing and instability. Null mutants with stable inheritance have been successfully generated in rice, arabidopsis, tomatoes, polar, white polar, apples, wheat, corn, soybean and other species through the CRISPR/Cas9 technology, but there have been no reports of genome editing by using CRISPR/Cas9 in *Medicago sativa.*

### SUMMARY

In order to use genome editing technology to modify the target genes in the autotetraploidplant *Medicago sativa,* and apply it to production practice, we developed a method forsite-specific mutagenesis by using a CRISPR/Cas9 system, thus achieving precise site-directed gene mutations in *Medicago sativa* with a mutation rate up to 52%, and generating null mutant plants with ideal phenotype and four mutated alleles (mutation rate reaches 12%). This method takes the lead in establishing an efficient targeted genome editing system in *Medicago sativa* and has a great significance in the researching and breeding of *Medicago sativa.* Therefore, the present disclosure is intended to provide a method for site-specific mutagenesis of *Medicago sativa* genes by using a CRISPR/Cas9 system.

The technical solution of the present disclosure is as below:
A method for site-specific mutagenesis of *Medicago sativa* genes by using a CRISPR/Cas9 system, wherein, the method comprises the following steps:
Step (1) Constructing a universal binary expression vector MsCRISPR/Cas9 that can be used for transforming *Medicago sativa* by *Agrobacterium tumefaciens;*
Step (2) Designing a CRISPR/Cas9-based sgRNA target site for a target gene in the Medicago sativa, and ligating the DNA fragment containing the guide sequence into the universal vector MsCRISPR/Cas9 in the Medicago sativato construct a vector MsCRISPR/Cas9::*target*;
Step (3)Transforming the *Medicago sativa* by the *Agrobacterium tumefaciens,* and introducing site-specific muta gene in the *Medicago sativa.*

Further, the *Medicago sativa* materials comprise wild varieties, local varieties, bred varieties, and introduced varieties.

Further, the target gene comprises any biologically functional genes or DNA sequences in the *Medicago sativa* genome.

Further, the complete sequence of the binary vector MsCRISPR/Cas9 for expressing the CRISPR/Cas9 system in the *Medicago sativais* as shown in SEQ ID NO.1.

The technology relies on the transformation of *Medicago sativa* by *Agrobacterium tumefaciens,* during which a T-DNA sequence containing a selection marker *Hpt* gene expression frame, a sgRNA expression frame and a *Cas9* gene expression frame is integrated into the *Medicago sativa* genome through transformation to generate transgene plants, wherein the transformed exogenous expression elements are expressed in *Medicago sativa* and thus function to generate plants with the gene mutated. An expression vector MsCRISPR/Cas9 is constructed for the transformation by *Agrobacterium.*

Preferably, the backbone vector of the binary expression vector MsCRISPR/Cas9 for expressing the CRISPR/Cas9 system in the *Medicago sativa* is a pCambia1300 vector; the pCambia1300 backbone vector contains a T-DNA region that is used to transform the plants; the T-DNA region comprises a left boundary repeat (LB repeat), a right boundary repeat (RB repeat) as well as a sequence between the left and right boundary sequences used for transformation; the sequence between the left and right boundary sequences of the T-DNA region used for transformation comprises a *Hpt* gene expression frame, a *Cas9* gene expression frame, an sgRNA expression frame; the *Hpt* gene expression frame comprises a CaMV 35S promoter (Enhanced) for driving the transcription of the *Hpt* gene, a CDS sequence of the *Hpt* gene and a CaMV poly A terminator for terminating the transcription of the *Hpt* gene; the *Cas9* gene expression frame comprises a 2xCaMV 35S promoter sequence for driving the transcription of the *Cas9* gene, a DNA sequence for expressing *Cas*9protein and a Nos terminator sequence for terminating the transcription of *Cas9* gene; the sgRNA expression frame comprises a MtU6 promoter from *Medicago truncatula* for driving the transcription of the sgRNA and a DNA sequence for expressing sgRNA.

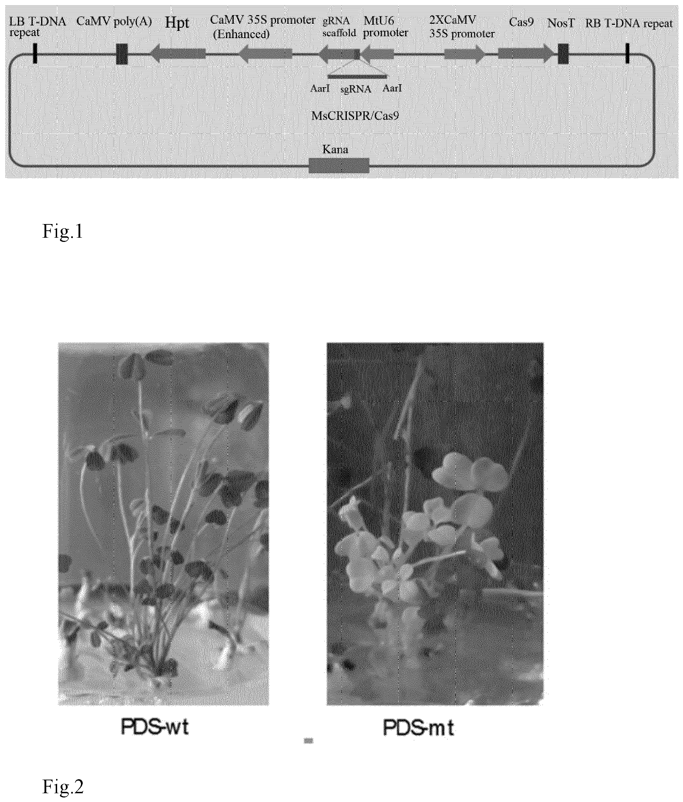

In this vector (Fig. 1), LB T-DNA repeat/RB T-DNA repeat represents the left and right boundary repeats of T-DNA; CaMV 35S promoter (Enhanced)/MtU6 promoter/2xCaMV 35S promoter represent promoter elements; *Hpt*/sgRNA/Cas9 represent gene sequences; CaMV poly (A)/NosT represent terminator sequences; AarI represents the insertion site of sgRNA guide sequence. As shown in Fig. 1: the *Medicago truncatula* U6 promoter is used to drive the expression of sgRNA; the 2xCaMV 35S promoter is used to drive the expression of *Cas9* gene; and the CaMV 35S promoter (Enhanced) is used to drive the expression of the selective marker gene *Hpt.*

Further, the sequence of the MtU6 promoter is as shown in SEQ ID NO.2.

Preferably, the DNA sequence for expressing sgRNA is as shown in SEQ ID NO.3; and the sequence from positions 1 to 30 in the DNA sequence for expressing sgRNA comprises two AarI restriction endonuclease recognition sites, which are used to construct the expression vector of site-specific mutagenesis for the target gene.

Further, the expression vector MsCRISPR/Cas9:: *target* is constructed as below:
Step (1) Designing a pair of oligonucleotide primers (18-24bp) according to the target site, which can form DNA fragments with linkers at both ends as below, and synthesize:
   5'-T T T G N₁₆₋₂₃-3'
   3'-C N₁₆₋₂₃C A A A-5';
Step (2) Annealing the above synthesized primer pair to form a complementary double-stranded DNA fragment with cohesive ends;
Step (3) Digesting the binary expression vector MsCRISPR/Cas9 with AarI restrictionendonuclease; dephosphorylating through reaction with CIP and then separating by electrophoresis in 0.8-1.2% of agarose gel, purifying the linear vector from gel;
Step (4) Ligating the complementary double-stranded DNA fragment with cohesive ends to the purified MsCRISPR/Cas9 linear vector by using a T4 DNA ligase in a water-bath pot at 16°C for 10-16 h;
Step (5) Transforming *Escherichia coli* DH5α competent cells with the ligated products by heat shock, coating on a LB plate medium containing 50 mg/L of kanamycin, and cultivating in an incubator at 37°C for 12-16 h;
Step (6) Selecting monoclonal bacterials and cultivating them in 1.5 mL LB liquid medium containing 50 mg/L of kanamycin, and culturing in a shaker at 37°C for 12-16 h;
Step (7) Sequecing the vector of monoclonal bacterials by using a sequencing primer MtU6-T-F, selecting proper monoclonal bacterials and cultivating them in 50-100 mL LB liquid medium containing 50 mg/L of kanamycin, culturingin a shaker at 37°C for 12-16 h, and extracting the plasmid.

Further, the sequence of the sequencing primer MtU6-T-F is as shown in SEQ ID NO.4.

Preferably, the *Medicago sativa* is transformed by using *Agrobacterium tumefaciens* to generate mutant plants through the following steps:
Step (1) Recovery of the strains of *Agrobacterium tumefaciens:* The *Agrobacterium* strainsafter being transformed by heat shock are coated in a YM solid medium, and cultured at 25-29°C for 24 h-48 h; monoclones are selected, inoculated in 30-50 mL YM liquid medium, and cultivated on a shaker at 100-220 r/min at 25-29°C for 24-48 h; the YM solid medium and the YM liquid medium both comprise 30-60 mg/L of kanamycin and 200-450 mg/L of rifampicin;
Step (2) Acquisition of *Medicago sativa* callus: The *Medicago sativa* callus is generated as below:
   Plump and well-colored *Medicago sativa* seeds are selected, soaked with 65-80% alcohol for 1-3minutes, washed with sterile water for 2-4 times, 0.5-2 minutes for each time; then soaked with 0.05-0.15% mercuric chloride and shaken by hands for 6-12 minutes, and washed with sterile water for 3-5 times; then inoculated on a MS solid medium, and germinated in light in an incubator or in a culture room for 7-16 days;
   Cotyledons and hypocotyls of the germinated seedlings are cut into small pieces and inoculated in a callus induction medium, and cultured in dark in an incubator or in a culture room at 25±1°C for 2-4 days; The ingredients of the callus induction medium are: SH basal medium + 1.0-3.0 mg/L 2,4-D (2,4-Difluorophenoxy acetic acid) + 0.1-0.3 mg/L KT (kinetin) + 0.1-0.5 mg/L casein hydrolysate + 15-40 g/L sucrose + 5.8-9 g/L agar; Preferably, the ingredients of the callus induction medium are: SH basal medium + 2 mg/L 2,4-D + 0.2 mg/L KT + 0.3 mg/L casein hydrolysate + 30 g/L sucrose + 8 g/L agar;
Step (3) Transformation of *Medicago sativa* callus with *Agrobacterium tumefaciens:* the *Medicago sativa* callus is transformed with *Agrobacterium* strains as below: the *Agrobacterium tumefaciens* strains recovered in step (1) are inoculated in 50-100 mL YM liquid medium (Sigma Co.) and cultured at 25-29°C on a shaker at 100-220 r/min until the OD value 260/280 is between 0.5-0.8; the bacterial solution is transferred into a 50 mL sterile centrifuge tube, and centrifuged at 0-8°C in a centrifuge at 3000-4500 r/min for 10-15 minutes; the supernatant is discarded, and a resuspension solution (the MS basal medium) is added for resuspension until the OD value 260/280 is between 0.5-0.8; acetosyringoneat 50-150 µmol/L is added; the resuspension solution is MS basal medium;the callus induced in step (2) is collected into a sterile triangular flask with a breathable and plastic sealing membrane, into which is poured the resuspended *Agrobacterium bacterial* solution, sealed with its own sealing membrane, and evacuated in a vacuum pump to 0.5 kpa for totally 0.5-1.5 h, during which the flask is shaken gently every 10-15 min; the triangular flask is taken out and shaken at 25-29°C on a shaker at 100-150 r/min for 0.5-1.5 h; all the bacterial solution is poured out, and the materials are spread out in a sterile culture dish containing filter papers to dry out;
Step (4) Co-cultivation of the *Medicago sativa* callus with *Agrobacterium tumefaciens:* The co-cultivation process is as below: The dried transformed materials are inoculated on a co-cultivation medium (spreading a piece of sterilized filter paper in the medium), and cultivated in dark in an incubator at 25±1°C for 2-5 days; the ingredients of the co-cultivation medium are: MS basal medium + 1-3 mg/L 2,4-D + 0.1-0.3 mg/L KT + 15-40 g/L sucrose + 5.8-9 g/L agar + 50-150 µmol/L acetosyringone; Preferably, the ingredients of the co-cultivation medium are: MS basal medium + 2 mg/L 2,4-D + 0.2 mg/L KT + 30 g/L sucrose + 8 g/L agar + 100 µmol/L acetosyringone;
Step (5) Screening cultivation of *Medicago sativa* callus: The process of screening cultivation is as below: The co-cultivated materials are inoculated on a screening medium, and cultivated in light in an incubator or in a culture room at 25±1°C for 30-60 days; the ingredients of the screening medium are: SH basal medium + 1-3 mg/L 2,4-D + 0.1-0.3 mg/L KT + 15-40 g/L sucrose + 5.8-9 g/L agar + 150-450 mg/L cefotaxime + 150-450 mg/L carbenicillin + 10-50 mg/L hygromycin; Preferably, the ingredients of the screening mediumare: SH basal medium + 2 mg/L 2,4-D + 0.2 mg/L KT + 30 g/L sucrose + 8 g/L agar + 250 mg/L cefotaxime + 250 mg/L carbenicillin + 15 mg/L hygromycin;
Step (6) Differentiation cultivation of *Medicago sativa* callus: The materials generated after screening cultivation are transferred onto a differentiation medium, and cultivated in light in an incubator or in a culture room at 25±1°C for 15-30 days; the ingredients of the differentiation medium are: UM basal medium + 0.5-5 g/L casein hydrolysate + 0.1-2 mg/L kinetin + 15-40 g/L sucrose + 5.8-9 g/L agar + 150-450 mg/L cefotaxime + 4-10 mg/L hygromycin; Preferably, the ingredients of the differentiation medium are: UM basal medium + 2 g/L casein hydrolysate + 0.4 mg/L kinetin + 30 g/L sucrose + 8 g/L agar + 250 mg/L cefotaxime + 5 mg/L hygromycin;
Step (7) Rooting cultivation of regenerated sprouts of *Medicago sativa:* The differentiated sprouts of 1-3 cm are transferred onto a rooting medium; the ingredients of the medium are: MS basal medium + 0.5-2 mg/L indolebutyric acid + 15-40 g/L sucrose + 5.8-9 g/L agar + 150-450 mg/L cefotaxime; Preferably, the ingredients of the rooting medium are: MS basal medium + 1 mg/L indolebutyric acid + 30 g/L sucrose + 8 g/L agar + 250 mg/L cefotaxime;
Step (8) Screening and genotyping of *Medicago sativa* mutants: The methods for screening and genotyping the *Medicago sativa* mutants comprise PCR-RE, T7E1 enzyme digestiongenotyping and targeted deep sequencing. The genotyping method is specifically as below:
   PCR-RE genotyping: DNA samples are extracted from the regenerated plants generated above, and specific primers are designed for the amplification of the target gene sequence. In the fragments generated from amplification, if there are restriction endonuclease recognition sites on the cutting sites of Cas9 protein, the mutated sequence cannot be digested by the restriction endonuclease due to the loss of recognition sites. Specific operations are: a. amplifying the fragments flanking the target sites of regenerated plants and control plants by using the specific primers; b. purifying the target fragments generated from amplification, and digesting the target fragments with target-specific restriction endonuclease; c. genotyping the digested products by agarose gel electrophoresis, and the results of electrophoresis are analyzed: after the amplification products of the regenerated seedlings are digested with restriction endonuclease, if there are bands in the electrophoretic bands of the same size as those in the control sequence without enzymatic digestion and there are no other bands, while the control sequence for restriction endonuclease digestion is cut into two smaller bands, it indicates that all alleles of the target gene are mutated and the regenerated seedlings are null mutants; after the amplification products of the regenerated seedlings are digested with restriction endonuclease, if there are bands in the electrophoresis results of the same size as those in the control sequence without enzymatic digestion, and if there are bands of the same size as those in the two smaller bands of the control sequence after digestion with restriction endonuclease, it indicates that only partial alleles of the target genes are mutated and wild-type alleles still exist; after the amplification products of the regenerated seedlings are digested with restriction endonuclease, if there are no bands in the electrophoretic bands of the same size as those in the control sequence without enzymatic digestion, and the bands after enzymatic digestion have the same size as those in the two smaller bands of the control sequence after digestion with restriction endonuclease, it indicates that no allele of the target genes is mutated; d. purifying the above bands which have not been cut, ligating them to pMD19-Tvector (Takara Co.) through TA cloning, transforming *Escherichia coli* competent cells and then selecting a batch of monoclones for sequencing, and analyzing the types of mutation on the target.

T7E1 genotyping: DNA samples are extracted from the regenerated plants generated above, and specific primers are designed for the amplification of the target gene sequence. In the target gene fragments generated from amplification, if there are no restriction endonuclease recognition sites on the cutting sites of Cas9 protein, the mutated plants are genotyped by T7E1 enzymatic digestion. Specific operations are: a. designing specific primers to amplify the sequence flanking the target sites; b. mixing the amplification products of wild-type and regenerated plants; c. denaturing and renaturing the mixed products; d. digesting the renatured products by using T7E1 enzyme (NEB Co.), and genotyping by agarose gel electrophoresis. Analysis of results: if there are mutations in the target genes of regenerated plants, after denaturation and renaturation, they will form a heteroduplex with the amplification products of wild-type plants, and the heteroduplex can be digested into two bands of ideal size by T7E1 enzyme; e. ligating the PCR products of regenerated seedlings which can be digested by T7E1 enzyme to a pMD19-T vector by TA cloning, transforming *Escherichia coli* competent cells and then selecting a batch of monoclones for sequencing, and analyzing the types of mutation on the target.

Genotyping by targeted sequencing: DNA samples are extracted from the regenerated plants generated above, and amplified the target site with specific amplification primers.PCR products are ligated into a pMD19-T vector by TA cloning, *Escherichia coli* competent cells is transformed and then a batch of monoclones are selected for targeted sequencing to analyze whether there are mutations on the target as well as the types of mutations.

At the same time, the present disclosure also claims an application of the method for site-specific mutagenesis of *Medicago sativa* genes by using a CRISPR/Cas9 system in *Medicago sativa* breeding.

The present disclosure has successfully established a system of CRISPR/Cas9-based genome editing technology in *Medicago sativa* for the first time, through which the target gene can be successfully mutated and null mutants with obvious phenotype can be generated. To realize genome editing in the autotetraploid plant *Medicago sativa,* on the basis of the existing platform of generating transgenic plants by transforming *Medicago sativa* with *Agrobacterium tumefaciens,* the present disclosure employs an *Agrobacterium* transforming method to integrate a T-DNA region of an expression frame containing the *Hpt* gene expression frame, an sgRNA expression frame and a *Cas9* protein expression frame into the *Medicago sativa* genome, wherein these elements are transcribed and translated in *Medicago sativa* cells and perform their function to generate mutants. The key point of expressing the CRISPR/Cas9 system in *Medicago sativa* is to find proper promoters, wherein the CaMV 35S promoter (Enhanced)for driving the transcription of *Hpt* gene and the 2xCaMV 35S promoter for driving the transcription of *Cas9* gene have been shown to drive the transcription of transgenes in *Medicago sativa;* the promoter for driving the transcription of sgRNA is generally U6 or U3 promoter, but there have been no report about the U6/U3 promoter in *Medicago sativa.* For driving the transcription of sgRNA efficiently, the MtU6 promoter from *Medicago truncatula* is selected.

Compared with the existing technology for downregulating the gene expression in *Medicago sativa* by means of RNAi, the present disclosure has the following benefits:
The present disclosure employs the CRISPR/Cas9 technology in the cross-pollinated autotetraploid plant *Medicago sativa* for the first time, and null mutantsare generated; the MtU6 promoter is firstly used in *Medicago sativa* for driving the transcription of sgRNA.; the MtU6 promoter is derived from Medicagotruncatula
1. Through the present disclosure, null mutants can be generated within one generation in the cross-pollinated autotetraploid *Medicago sativa,* which has great value for accelerating the breeding and researching of *Medicago sativa.*
2.The present disclosure firstly establishes a CRISPR/Cas9 genome editing platform in *Medicago sativa* based on transformation by *Agrobacterium tumefaciens,* through which the target genes can be mutated directly, thus making the technology more complete and stable.
3. Compared with natural mutations and mutagens-induced mutations, the present disclosure introduces mutations with higher mutation efficiency, the mutation rate being up to 52% (wherein, the mutation rate of null mutants reaches 12%).
4.The technology opens up a new field for genome editing in *Medicago sativa,* laying a technical foundation for the subsequent simultaneous editing of multiple genes, large fragment deletion of chromosome, and precise insertion of exogenous gene; at the same time, Cas9 protein can be mutated and inactivated to be dCas9/nCas9 that can be coupled to protein domains with other functions to achieve the expression upregulation/downregulation, methylation/demethylation, and base substitution of the target gene.

### BRIEF DESCRIPTION OF THE DRAWINGS

Fig. 1 is the structure diagram of MsCRISPR/Cas9 expression vector;
Fig. 2 shows the wild and mutant plants of *PDS* gene generated by transforming the *Medicago sativa* with MsCRISPR/Cas9::*PDS* vector which contains the target of *PDS* gene;
Fig. 3 shows the sequencing results of PCR products of *PDS* gene of wild and mutant *Medicago sativa* plants;
Fig. 4 shows the results of PCR-RE analyses for identifying mutants of *PALM1* gene;
Fig. 5 shows the leaf morphologies of wild-type and mutant-type plants of the *PALM1* gene;
Fig. 6 shows the sequencing results of PCR products of *PALM1* gene of wild and mutant *Medicago sativa* plants.

### DETAILED DESCRIPTION

The technical solution of the present disclosure will be further described below in detail in combination with specific examples, but the present disclosure is not limited to the following technical solution.

### Example 1 Mutation of phytoene desaturase encoding gene PDS in Medicago sativa by means of a CRISPR/Cas9 system

Phytoene desaturase (*PDS*) is a major rate-limiting enzyme in synthesis of carotenoid, which can catalyze colorless C40 phytonee to generate ζ-carotene, neurosporene, lycopene, 3, 4-dehydrolycopene, 3, 4, 3'4'-dehydrolycopene or 3, 4-dehydroneurosporene, etc. The mutant plants of this gene show albino phenotype, which is easy to observe. Using this gene as the target gene in plants is a convenient method to determine whether the gene knockout system works as well as to evaluate the working efficiency.

According to the *PDS* sequence in *Medicago truncatula* (a proximal species of *Medicago sativa*), a primer set was designed to amplify a *PDS* gene sequence of *Medicago sativa* and the *PDS* gene was sequenced; according to the resulting *PDS* gene sequence and with reference to the case in *Medicago truncatula,* a target site was designed; the target site was ligated into a site of the vector MsCRISPR/Cas9 to generate a vector for knocking out the *PDS* gene; the vector was transformed into the explant of *Medicago sativa* in the above transforming protocol by *Agrobacterium tumefaciens,* generating the regenerated plants (Fig. 2). Wherein, the construction of the expression vector, the preparation of *Agrobacterium* strains, the transformation by means of *Agrobacterium,* and the screening and genotyping of mutants were as below:
First. The expression vector was constructed as below:
1. Designing a pair of oligoprimers (18-24bp) according to the target site, which can form DNA fragments with linkers at both ends as below, and synthesize:
   5'-T T T G N₁₆₋₂₃-3'
   3'-C N₁₆₋₂₃C A A A-5';
2.Annealing the above synthesized primer pair;
3.Digesting the vector MsCRISPR/Cas9 with AarI restriction enzyme; after reaction with CIP, separating in 0.8-1.2% of agarose gel, cutting the gel and purifying the linear vector;
4.Ligating the annealed and phosphorylated DNA fragments to the gel recycled MsCRISPR/Cas9 linear vector by using a T4 DNA ligase in a water-bath pot at 16°C for 10-16h;
5. Transforming *Escherichia coli* DH5α competent cells with the ligated products by heat shock, coating on a LB plate medium containing 50 mg/L of kanamycin, and cultivating in an incubator at 37°C for 12-16 h;
6. Selecting monoclones in 1.5 ml LB liquid medium containing 50 mg/L of kanamycin, and culturingin a shaker at 37°C for 12-16 h;
7. Sequencing the vector of monoclonal bacterials by using a sequencing primer, selecting proper monoclonal bacterials and cultivating them in 50-100 mL LB liquid medium containing 50 mg/L of kanamycin, culturingin a shaker at 37°C for 12-16 h, and extracting the vectors.

The sequencing primer is as below:
MtU6-T-F:GGCATGCAAGCTTATCGATAC.

### Second. Preparation of Agrobacterium strains

The above expression vector was transformed into *Agrobacterium tumefaciens* strain EHA105 competent cells by electroporation, recovered and coated in a YM medium (with 50 mg/L of kanamycin and 250 mg/L of rifampicin), cultured at 28°C for 24-48 h; monoclones were selected and inoculated in 30-50 mL YM liquid medium (with 50 mg/L of kanamycin and 250 mg/L of rifampicin), shaken at 28°C in a shaker at 200 r/min for 24-48 h.

### Third. Transformation by means of Agrobacterium

A DNA sequence for expressing the CRISPR/Cas9 system and the Hpt protein that is resistant to the plant screening agent hygromycin was integrated into the genome of *Medicago sativa* by means of transformation with *Agrobacterium,* so that the expression products of these elements functioned to generate positive plants with the target gene silenced through screening. Specific transforming operations were as below:
1. Germination of *Medicago sativa* seeds: Plump and well-colored *Medicago sativa* seeds were selected, soaked with 75% alcohol for 2 minutes, washed with sterile water for two times, 1 minute for each time; then soaked with 0.1% mercuric chloride and shaken by hands for 10 minutes, and washed with sterile water for five times. The sterilized seeds were spread out in a large sterile culture dish containing filter papers, air-dried, inoculated on a MS solid medium, and germinated in light in an incubator or in a culture room for 7-14 days;
2. Induction of callus: Cotyledons and hypocotyls of the germinated seedlings were cut into small pieces with a sterile scalpel and inoculated on a callus induction medium, and cultured in dark in an incubator or in a culture room at 25±1°C for 3 days. The ingredients of the callus induction medium were: SH basal medium+ 2 mg/L 2,4-D+ 0.2 mg/L KT+ 0.3 mg/L casein hydrolysate + 30 g/L sucrose + 8 g/L agar;
3.Infection: 1-2 days in advance, the prepared *Agrobacterium tumefaciens* strains were inoculated in 50-100 mL YM liquid medium and cultured at 28°C on a shaker at 200 r/min until the OD value 260/280 was between 0.5-0.8; the bacterial solution was transferred into a 50 mL sterile centrifuge tube, and centrifuged at 4°C in a centrifuge at 4000 r/min for 12 minutes; the centrifuge tube was taken out, the supernatant was discarded, and a resuspension solution was added for resuspension until the OD value 260/280 was between 0.5-0.8; Corresponding volume of acetosyringone at 100 µmol/ml was added according to adding 100 µmol acetosyringone per liter; the resuspension solutionwas MS liquid medium (MS + 30 g/L sucrose); the explants induced in step 2 were collected into a 100 ml sterile triangular flask with a breathable and plastic sealing membrane, into which was poured a suitable amount of the resuspendedbacterial solution (as long as covering the materials); the triangular flask was sealed with its own sealing membrane, and evacuated in a vacuum pump to 0.5 kpa for totally 1 h, during which the flask was shaken gently every 15 min; the triangular flask was taken out and shaken at 28°C on a shaker at 120 r/min for 1 h; all the bacterial solution was poured out, and the materials were spread out in a sterile culture dish containing filter papers to dry out;
4. Co-cultivation: The dried transformed materials were inoculated on a co-cultivation medium (spreading a piece of sterile filter paper in the medium); the ingredients of the co-cultivation medium were: MS basal medium + 2 mg/L 2,4-D + 0.2 mg/L KT + 30 g/L sucrose + 8 g/L agar + 100 µmol/L acetosyringone. They were cultivated in dark in an incubator or in a culture room at 25±1°C for 3 days.
5. Screening: The co-cultivated materials were inoculated on a screening medium; the ingredients of the screening medium were: SH basal medium + 2 mg /L 2,4-D + 0.2 mg/L KT + 30 g/L sucrose + 8 g/L agar + 250 mg/L cefotaxime + 250 mg/L carbenicillin + 15 mg/L hygromycin; they were cultivated in light in an incubator or in a culture room at 25±1°C for 30-60 days;
6. Differentiation: The recovered materials were transferred onto a differentiation medium; the ingredients of the differentiation medium were: UM basal medium + 2 g/L casein hydrolysate + 0.4 mg/L kinetin + 30 g/L sucrose + 8 g/L agar + 250 mg/L cefotaxime + 5 mg/L hygromycin;
7. Rooting: The differentiated sprouts of 1-3cm were transferred onto a rooting medium; the ingredients of the rooting medium were: MS basal medium + 1 mg/L indolebutyric acid + 30 g/L sucrose + 8 g/L agar + 250 mg/L cefotaxime.

### Fourth. Screening and genotyping of mutants

Genotyping by targeted deep sequencing: DNA samples were extracted from the regenerated plants generated above, and amplified *PDS* sequence flanking the target site with specific amplification primers. PCR products were ligated into a pMD19-T vector by TA cloning, the *Escherichia coli* strain DH5α was transformed and then a batch of monoclones were selected for targeted deep sequencing to analyze whether there were mutations on the target as well as the types of mutations.

### We found through experiments and genotyping:

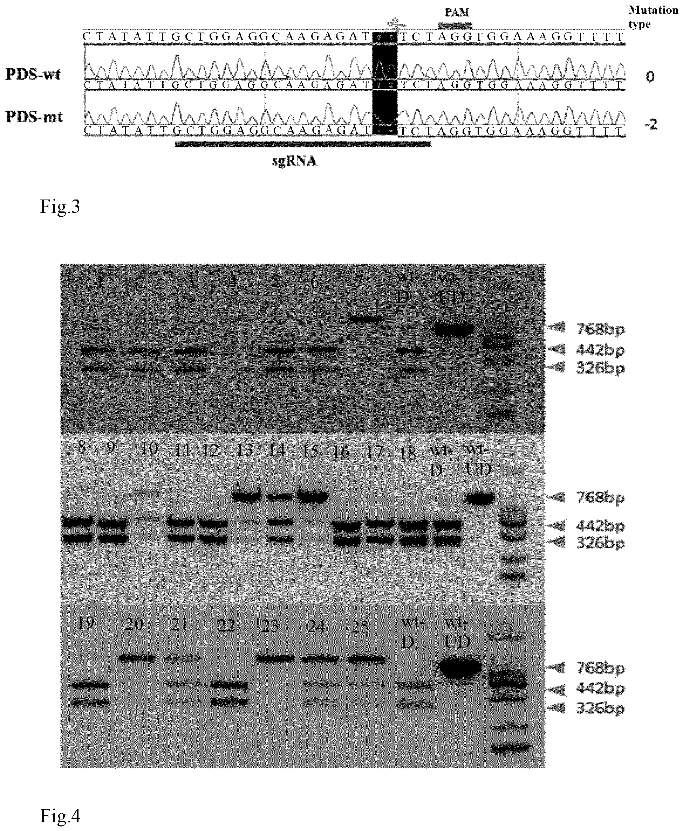

As can be seen by phenotypic observation, an albino seedling was generated (Fig. 2). DNA samples were extracted from the albino seedling and the *PDS* gene sequence was amplified. The sequencing results (Fig. 3) showed that: there was a 2bp base deletion at the target site of the plant, and the base deletion site was the cleavage site of Cas9 protein.

Fig. 2 showed that: wild-type (*PDS-wt*); mutant-type (*PDS-*mt)*.* Wild-type plants showed normal green phenotype; while mutant plants showed white phenotype. The results were consistent with the phenotype of plantsgenerated by *PDS* gene knockout mediated by CRISPR/Cas9 in white poplar, apple and other species, indicating that the resulting albino seedling was probably caused by the *PDS* gene mutation mediated by the introduced CRISPR/Cas9 system.

Fig. 3 showed that: As can be seen from the sequencing results, compared with wild-type plants, there was a 2 bp base deletion at the target site of the *PDS* mutant plants, and the mutation was located at the cleavage site of Cas9 protein, indicating that the albino seedling of *Medicago sativa* in Fig. 2 is caused by the 2 bp base deletion occurring in the process of gene repairing resulting from the cleavage of *PDS* gene target site by CRISPR/Cas9.

### Example 2 PALM1 gene mutation in Medicago sativa

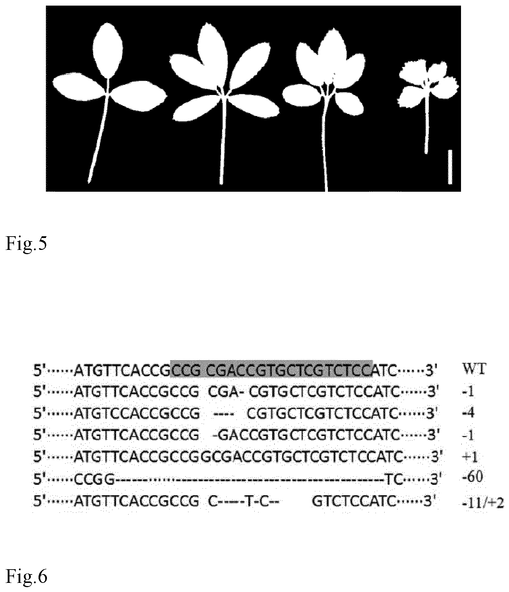

This example is different from example 1 in that:
According to the *PALM1* sequence in *Medicago truncatula* (a proximal species of Medicago sativa), a primer set was designed to amplify a *MsPALM1* gene sequence of *Medicago sativa* and the *MsPALM1* gene was sequenced; according to the resulting *MsPALM1* gene sequence, a target site was designed; the target site was ligated into a site of the vector MsCRISPR/Cas9 to generate a vector MsCRISPR/Cas9::*PALM1* for knocking out the *MsPALM1* gene; the vector was transformed into the explant of *Medicago sativa* in the above transforming protocol by *Agrobacterium tumefaciens,* generating the regenerated plants. The genotyping method was PCR-RE, in which genome DNA samples were extracted from the regenerated plants generated above, and amplified *MsPALM1* gene flanking the target site with specific amplification primers; and PCR products were digested with BstUI restriction endonuclease to screen mutant strains (Fig. 4); and the mutations were confirmed by phenotype observation (Fig. 5) and targeted deep sequencing (Fig. 6).

Fig. 4 showed that: As shown by PCR-RE genotyping, in the sampled 25 *Medicago sativa* regenerated plants, there were 13 mutant plants (there were bands that could not be digested with restriction endonuclease BstUI at 768bp), wherein there were 10 heterozygous mutant plants (besides the bands that could not be digested with restriction endonuclease BstUI at 768bp, there were also two bands generated after digestion with restriction endonuclease BstUI, which are located at 442 bp and 326bp, respectively), and 3 null mutants (there were only the bands that could not be digested with restriction endonuclease BstUI at 768bp); the mutation rate reached 52%, wherein the mutation rate of null mutants reached 12%.

Fig. 5 showed that, in the sampled 25 *Medicago sativa* regenerated plants, the compound leaves of three plants (No. 2, 3, 4 from left to right) showed obvious phenotypic changes. Compared with the control plant (No. 1 from left to right), the number of leaflets in the compound leaves of the mutant plants changed from three to five. Wherein, the mutant plant No. 3 (i.e., No. 20 in Fig. 4) showed weak bands that could be digested with BstUI restriction endonuclease in PCR-RE results, and this may be due to that the plants of T0 generation were chimera.

Fig. 6 showed that, a part of plants screened from Fig. 4 were selected to subject to targeted deep sequencing for the *MsPALM1* gene, and mutations have been successfully introduced into the prE determined target of CRISPR/Cas9 on the *MsPALM1* gene.

The above results showed that site-specific mutagenesis has been achieved successfully in *Medicago sativa* by the CRISPR/Cas9 technology.

## Claims

1. A method for site-specific mutagenesis of *Medicago sativa* genes by using a CRISPR/Cas9 system, wherein, the method comprises the following steps:
Step (1) Constructing a universal binary expression vector MsCRISPR/Cas9 that can be used for transforming *Medicago sativa* by *Agrobacterium tumefaciens;*
Step (2) Designing a CRISPR/Cas9-based target site for a target gene in the *Medicago sativa,* and ligating the DNA fragment containing the guide sequence of the target site into the universal vector MsCRISPR/Cas9 to construct a vector MsCRISPR/Cas9: :*target;*
Step (3) Transforming the *Medicago sativa* by the *Agrobacterium tumefaciens,* and introducing site-specific mutations into the target gene.

2. The method for site-specific mutagenesis of *Medicago sativa* genes by using a CRISPR/Cas9 system according to claim 1, wherein, the *Medicago sativa* materials comprise wild varieties, local varieties, bred varieties, and introduced varieties.

3. The method for site-specific mutagenesis of *Medicago sativa* genes by using a CRISPR/Cas9 system according to claim 1, wherein, the target gene comprises any biologically functional genes or DNA sequences in the *Medicago sativa* genome.

4. The method for site-specific mutagenesis of *Medicago sativa* genes by using a CRISPR/Cas9 system according to claim 1, wherein, the complete sequence of the binary expression vector MsCRISPR/Cas9 for expressing the CRISPR/Cas9 system in the *Medicago sativais* as shown in SEQ ID NO.1.

5. The method for site-specific mutagenesis of *Medicago sativa* genes by using a CRISPR/Cas9 system according to claim 1, wherein, the backbone vector of the binary expression vector MsCRISPR/Cas9 for expressing the CRISPR/Cas9 system in the *Medicago sativa* is a pCambia1300 vector; the pCambia1300 backbone vector contains a T-DNA region that is used to transform the plants; the T-DNA region comprises a left boundary repeat (LB repeat), a right boundary repeat (RB repeat) as well as a sequence between the left and right boundary sequences used for transformation; the sequence between the left and right boundary sequences of the T-DNA region used for transformation comprises a *Hpt* gene expression frame, a *Cas9* gene expression rame, an sgRNA expression frame; the *Hpt* gene expression frame comprises a CaMV 35S promoter (Enhanced) sequence for driving the transcription of the *Hpt* gene, a CDS sequence for expressing the *Hpt* gene and a CaMV poly A terminator sequence for terminating the transcription of the *Hpt* gene; the *Cas9* gene expression frame comprises a 2xCaMV 35S promoter sequence for driving the transcription of the *Cas9* gene, a DNA sequence for expressing Cas9protein and a Nos terminator sequence for terminating the transcription of *Cas9* gene; the sgRNA expression frame comprises a MtU6 promoter from *Medicago truncatulaa Medicagotruncatula* MtU6 promoter sequence for driving the transcription of the sgRNAsequence and aDNA sequence for expressing sgRNA.

6. The method for site-specific mutagenesis of *Medicago sativa* genes by using a CRISPR/Cas9 system according to claim 5, wherein, the sequence of the MtU6 promoter is as shown in SEQ ID NO.2.

7. The method for site-specific mutagenesis of *Medicago sativa* genes by using a CRISPR/Cas9 system according to claim 5, wherein, the DNA sequence for expressing sgRNA is as shown in SEQ ID NO.3, and the DNA sequence from positions 1 to 30 comprises two AarI restriction endonuclease recognition sites, which are used to construct the expression vector of site-specific mutagenesis for the target gene.

8. The method for site-specific mutagenesis of *Medicago sativa* genes by using a CRISPR/Cas9 system according to any one of claims 1-7, wherein, the expression vector MsCRISPR/Cas9::*target* is constructed as below:
Step (1) Designing a pair of oligoprimers18-24bp according to the target site, which can form DNA fragments with linkers at both ends as below, and synthesize:
5'-T T T G N₁₆₋₂₃-3'
3'-C N₁₆₋₂₃C A A A-5';
Step (2) The above synthesized primer pair is annealed to form a double-stranded DNA fragment of complementary oligoprimer dimers with cohesive ends;
Step (3) Digesting the binary expression vector MsCRISPR/Cas9 withAarI restriction endonuclease; dephosphorylating with calf alkaline phosphase CIP and then separating by electrophoresis in 0.8-1.2% of agarose gel, purifying the linear vector from gel;
Step (4) Ligating the complementary double-stranded DNA fragment with cohesive ends after annealing and dephosphorylation to the purified MsCRISPR/Cas9 linear vector by using a T4 DNA ligase in a water-bath pot at 16°C for 10-16 h;
Step (5) Transforming *Escherichia coli* DH5α competent cells with the ligated products by heat shock, coating on a LB plate medium containing 50 mg/L of kanamycin, and cultivating in an incubator at 37°C for 12-16 h;
Step (6) Selecting single colonies and cultivating them in 1.5 mL LB liquid medium containing 50 mg/L of kanamycin, and culturingin a shaker at 37°C for 12-16 h;
Step (7)Sequencing the vector of monoclonal bacterials by using a sequencing primer MtU6-T-F, selecting proper monoclonal bacterials and cultivating them in 50-100 mL LB liquid medium containing 50 mg/L of kanamycin, culturingin a shaker at 37°C for 12-16 h, and extracting the vectors.

9. The method for site-specific mutagenesis of *Medicago sativa* gene by using a CRISPR/Cas9 system according to claim 6, wherein, the sequence of the sequencing primer MtU6-T-F is as shown in SEQ ID NO.4.

10. The method for site-specific mutagenesis of *Medicago sativa* genes by using a CRISPR/Cas9 system according to claim 1, wherein, the *Medicago sativa* is transformed by using *Agrobacteriu mtumefaciens* to generate mutant plants through the following steps:
Step (1) Recovery of the strains of *Agrobacteri umtumefaciens:* The Agrobacterium strains after being transformed by heat shock are coated in a YM solid medium, and cultured at 25-29°C for 24-48 h; monoclones are selected, inoculated in 30-50 mL YM liquid medium, and shaken on a shaker at 100-220 r/min at 25-29°C for 24-48 h; the YM solid medium and the YM liquid medium both comprise 30-60 mg/L of kanamycin and 200-450 mg/L of rifampicin;
Step (2) Acquisition of *Medicago sativa* callus: The *Medicago sativa* callus is generated as below:
Plump and well-colored *Medicago sativa* seeds are selected, soaked with 65-80% alcohol for 1-3 minutes, washed with sterile water for 2-4 times, 0.5-2 minutes for each time; then soaked with 0.05-0.15% mercuric chloride and shaken by hands for 6-12 minutes, and washed with sterile water for 3-5 times; then inoculated on a MS solid medium, and germinated in light in an incubator or in a culture room for 7-16 days;
Cotyledons and hypocotyls of the germinated seedlings are cut into small pieces and inoculated in a callus induction medium, and cultured in dark in an incubator or in a culture room at 25±1°C for 2-4 days; The ingredients of the callus induction medium are: SH basal medium + 1.0-3.0 mg/L 2,4-Difluorophenoxy acetic acid + 0.1-0.3 mg/L kinetin + 0.1-0.5 mg/L casein hydrolysate + 15-40 g/L sucrose + 5.8-9 g/L agar; Preferably, the ingredients of the callus induction medium are: SH basal medium + 2 mg/L 2,4-Difluorophenoxy acetic acid + 0.2 mg/L kinetin + 0.3 mg/L casein hydrolysate + 30 g/L sucrose + 8 g/L agar;
Step (3) Infection of *Medicago sativa* callus with *Agrobacteriumtumefaciens:* the *Medicago sativa* callus is transformed with *Agrobacterium* strains as below: 1-2 days in advance, the *Agrobacteriumtumefaciensstrains* recovered in step (1) are inoculated in 50-100 mL YM liquid medium and cultured at 25-29°C on a shaker at 100-220 r/min until the OD value 260/280 is between 0.5-0.8; the bacterial solution is transferred into a 50 mL sterile centrifuge tube, and centrifuged at 0-8°C in a centrifuge at 3000-4500 r/min for 10-15 minutes; the supernatant is discarded, and a resuspension solution is added for resuspension until the OD value 260/280 is between 0.5-0.8; acetosyringone at 50-150 µmol/L is added; the resuspension solution is MS basal medium + 30 g/L sucrose; the callus induced in step(2) is collected into a sterile triangular flask with a breathable and plastic sealing membrane, into which is poured the resuspended *Agrobacterium* bacterial solution, sealed with its own sealing membrane, and evacuated in a vacuum pump to 0.5 kpa for totally 0.5-1.5 h; the triangular flask is taken out and shaken at 25-29°C on a shaker at 100-150 r/min for 0.5-1.5 h; all the bacterial solution is poured out and dried in the air;
Step (4) Co-cultivation of the *Medicago sativa* callus with *Agrobacteriumtumefaciens:* The co-cultivation process is as below: The dried transformed materials are inoculated in a co-cultivation medium (spreading a piece of sterilized filter paper in the medium), and cultivated in dark in an incubator at 25±1°C for 2-5 days; the ingredients of the co-cultivation medium are: MS basal medium + 1-3 mg/L 2,4-Difluorophenoxy acetic acid + 0.1-0.3 mg/L kinetin + 15-40 g/L sucrose + 5.8-9 g/L agar + 50-150 µmol/L acetosyringone; Preferably, the ingredients of the co-cultivation medium are: MS basal medium + 2 mg/L 2,4-Difluorophenoxy acetic acid + 0.2 mg/L kinetin + 30 g/L sucrose + 8 g/L agar + 100 µmol/L acetosyringone;
Step (5) Screening cultivation of *Medicago sativa* callus: The process of screening cultivation is as below: The co-cultivated materials are inoculated in a screening medium, and cultivated in light in an incubator or a culture room at 25±1°C for 30-60 days; the ingredients of the screening mediumare: SH basal medium + 1-3 mg/L 2,4-Difluorophenoxy acetic acid + 0.1-0.3 mg/L kinetin + 15-40 g/L sucrose + 5.8-9 g/L agar + 150-450 mg/L cefotaxime + 150-450 mg/L carbenicillin + 10-50 mg/L hygromycin; Preferably, the ingredients of the screening medium are: SH basal medium + 2 mg/L 2,4-Difluorophenoxy acetic acid + 0.2 mg/L kinetin + 30 g/L sucrose + 8 g/L agar + 250 mg/L cefotaxime + 250 mg/L carbenicillin + 15 mg/L hygromycin;
Step (6) Differentiation cultivation of *Medicago sativa* callus: The materials generated after screening cultivation are transferred into a differentiation medium, and cultivated in light in an incubator or a culture room at 25±1°C for 15-30 days; the ingredients of the differentiation medium are: UM basal medium + 0.5-5 g/L casein hydrolysate + 0.1-2 mg/L kinetin + 15-40 g/L sucrose + 5.8-9 g/L agar + 150-450 mg/L cefotaxime + 4-10 mg/L hygromycin; Preferably, the ingredients of the differentiation medium are: UM basal medium + 2 g/L casein hydrolysate + 0.4 mg/L kinetin + 30 g/L sucrose + 8 g/L agar + 250 mg/L cefotaxime + 5 mg/L hygromycin;
Step (7) Rooting cultivation of regenerated sprouts of *Medicago sativa:* The differentiated sprouts of 1-3 cm are transferred into a rooting medium; the ingredients of the mediumare: MS basal medium + 0.5-2 mg/L indolebutyric acid + 15-40 g/L sucrose + 5.8-9 g/L agar + 150-450 mg/L cefotaxime; Preferably, the ingredients of the rooting mediumare: MS basal medium + 1 mg/L indolebutyric acid + 30 g/L sucrose + 8 g/L agar + 250 mg/L cefotaxime;
Step (8) Screening and genotyping of *Medicago sativa* mutants: The methods for screening and genotyping the *Medicagosativa* mutants comprise PCR-RE genotyping, T7E1 enzyme digestiongenotyping and targeted deep sequencing.

11. An application of the method for site-specific mutagenesis of *Medicago sativa* genes by using a CRISPR/Cas9 system according to any one of claims1-10 in *Medicago sativa* breeding and *Medicago sativa* breeding.
